# EUROPÄISCHE PATENTSCHRIFT

(11) **EP 1 315 607 B1**
(45) Veröffentlichungstag und Bekanntmachung des Hinweises auf die Patenterteilung: **07.04.2004**
(21) Anmeldenummer: 02753040.1
(22) Anmeldetag: 02.05.2002
(51) Int. Cl.: B29C 43/18, B29C 37/02, A61M 5/315, B29C 43/42

(54) **PHARMAZEUTISCHER SPRITZENKOLBEN SOWIE VERFAHREN UND VORRICHTUNG ZU DESSEN HERSTELLUNG**
PHARMACEUTICAL SYRINGE PISTON AND METHOD AND DEVICE THEREFOR
PISTON DE SERINGUE PHARMACEUTIQUE ET PROCEDE ET DISPOSITIF DE FABRICATION DE CELUI-CI

(30) Priorität: 11.05.2001 DE 10122959
(43) Veröffentlichungstag der Anmeldung: 04.06.2003
(73) Patentinhaber: West Pharmaceutical Services Deutschland Gmbh & Co. KG, 52249 Eschweiler (DE)
(72) Erfinder: BRINKHUES, Jürgen, 52072 Aachen (DE)
(74) Vertreter: Maucher, Wolfgang, Dipl.-Ing.
(86) Internationale Anmeldenummer: PCT/EP2002/004831
(87) Internationale Veröffentlichungsnummer: WO 2002/092312

(56) Entgegenhaltungen:
- EP-A- 0 148 426
- GB-A- 593 531
- US-A- 5 904 891
- US-A- 6 165 402

## Beschreibung

Die Erfindung betrifft ein Verfahren zum Herstellen eines im wesentlichen aus Gummi oder dergleichen elastomerem Werkstoff bestehenden pharmazeutischen Kolbenstopfens, der eine Aufnahmehöhlung zum Verbinden mit einer Kolbenstange oder dergleichen Bewegungsübertragungselement und einen von einem fluorierten Polymerfilm oder dergleichen Inertfilm kappenförmig umschlossenen Kolbenabschnitt hat, der in Gebrauchsstellung dem Inhalt eines Spritzen- oder Karpulenzylinders zugewandt ist und am Außenumfang mit seinem Inertfilm an dem Spritzen- oder Karpulenzylinder anliegt, welcher Kolbenstopfen benachbart zu diesem Kolbenabschnitt einen unbeschichteten, in Gebrauchsstellung an dem Spritzen- oder Karpulenzylinder anliegenden Dichtabschnitt hat, wobei in einem Formungsschritt eine aus einem unvulkanisierten Gummifell und einem fluorierten Polymerfilm oder dergleichen Inertfilm bestehende Schichtanordnung in ein Formwerkzeug eingelegt und unter Einwirkung von Druck und Wärme zu einem Kolbenstopfenteil geformt wird, wobei das Gummifell vulkanisiert und untrennbar mit dem Inertfilm verbunden wird.

Außerdem betrifft die Erfindung eine Vorrichtung zur Herstellung von im wesentlichen aus Gummi oder dergleichen elastomerem Werkstoff bestehenden pharmazeutischen Kolbenstopfen, die eine Aufnahmehöhlung zum Verbinden mit einer Kolbenstange oder dergleichen Bewegungsübertragungselement und einen in Gebrauchsstellung dem Inhalt eines Spritzen- oder Karpulenzylinders zugewandten, von einem fluorierten Polymerfilm oder dergleichen Inertfilm kappenförmig umschlossenen Kolbenabschnitt haben, der in Gebrauchsstellung am Außenumfang mit seinem Inertfilm an dem Spritzen- oder Karpulenzylinder anliegt, welcher Kolbenstopfen benachbart zu diesem Kolbenabschnitt einen unbeschichteten, in Gebrauchsstellung an dem Spritzen- oder Karpulenzylinder anliegenden Dichtabschnitt hat, mit einem Formwerkzeug zum Formen des Kolbenstopfens aus einer aus einem unvulkanisierten Gummifell und einem fluorierten Polymerfilm oder dergleichen Inertfilm bestehenden Schichtanordnung, wobei das Formwerkzeug zumindest eine erste Formplatte und eine damit zusammenwirkende zweite Formplatte hat, die relativ zueinander in eine Schließund eine Offenstellung bewegbar sind, sowie mit einer Stanzvorrichtung zum Abtrennen eines im Bereich der Trennebene des Formwerkzeugs verbleibenden, seitlich über den Kolbenstopfen überstehenden Schichtanordnungsflansches von dem Kolbenstopfen.

Des Weiteren bezieht sich die Erfindung auf einen pharmazeutischen Kolbenstopfen, der einen Grundkörper aus Gummi oder dergleichen elastomerem Werkstoff aufweist, der eine Aufnahmehöhlung zum Verbinden mit einer Kolbenstange oder dergleichen Bewegungsübertragungselement und einen von einem fluorierten Polymerfilm oder dergleichen Inertfilm umschlossenen Kolbenabschnitt hat, der in Gebrauchsstellung dem Inhalt eines Spritzen- oder Karpulenzylinders zugewandt ist und am Außenumfang mit seinem Inertfilm an dem Spritzen- oder Karpulenzylinder anliegt, wobei der Kolbenstopfen benachbart zu diesem Kolbenabschnitt einen unbeschichteten, in Gebrauchsstellung an dem Spritzen- oder Karpulenzylinder anliegenden Dichtabschnitt hat, der wenigstens eine an seinem Außenumfang umlaufende Dichtlippe aufweist.

In vorgefüllten Spritzen- oder Karpulenzylindern aufzubewahrende, in der Regel flüssige oder pulverförmige Medikamente oder pharmazeutische Zubereitungen stellen unterschiedliche Anforderungen an den zu verwendenden Kolbenstopfen. So ist eine chemische oder biologische Verträglichkeit mit dem Inhalt des Spritzen- oder Karpulenzylinders gefordert, was einerseits bedeutet, daß in dem Werkstoff des Kolbenstopfens enthaltene Stoffe nicht in das in dem Spritzen- oder Karpulenzylinder befindliche Medikament gelangen und dieses verunreinigen oder in seiner therapeutischen Wirkung verändern dürfen. Andererseits sollen aber auch aus dem Medikament keine Wirkstoffe in den Kolbenstopfen eindringen können, da dann mit zunehmender Aufbewahrungsdauer die Konzentration des Wirkstoffs in dem Medikament abnimmt. Schließlich können Stoffe, die aus dem Medikament in den Kolbenstopfen eindringen, aber auch die funktionellen Eigenschaften des Kolbenstopfens verändern. Darüber hinaus soll der Kolbenstopfen das Innere des Spritzenoder Karpulenzylinders auch gas-, flüssigkeits- und keimdicht abdichten.

Aus EP 0 148 426 A2 ist bereits ein im wesentlichen aus Gummi bestehender Kolbenstopfen bekannt, der einen in Gebrauchsstellung dem Inhalt des Spritzenzylinders zugewandten, von einem Inertfilm kappenförmig umschlossenen Kolbenabschnitt hat, der an seinem Außenumfang eine umlaufende, mit dem Inertfilm beschichtete Dichtlippe hat, die an dem Spritzenzylinder anliegt. Dabei soll der Inertfilm einen direkten Kontakt zwischen dem Inhalt des Spritzenzylinders und dem damit unverträglichen Gummiwerkstoff verhindern. Um die Abdichtung des Kolbenstopfens gegen den Spritzenzylinder zu verbessern, hat der Kolbenstopfen benachbart zu dem beschichteten Kolbenabschnitt einen unbeschichteten Dichtabschnitt, der an seinem Außenumfang zwei parallel zueinander angeordnete, umlaufende Dichtlippen aufweist. Diese unbeschichteten Dichtlippen liegen in Gebrauchsstellung an dem Spritzenzylinder an. Zwischen den beiden Dichtlippen des Dichtabschnitts sowie zwischen der Dichtlippe des beschichteten Kolbenabschnitts und der dazu benachbarten Dichtlippe des Dichtabschnitts ist jeweils ein Rücksprung vorgesehen, durch den die Dichtlippen voneinander beabstandet sind.

Für vorgefüllte Spritzensysteme, bei denen das Medikament über einen längeren Zeitraum in der Spritze gelagert wird, sind die Kolbenstopfen jedoch nur bedingt geeignet. Die mit dem Inertfilm beschichtete erste Dichtlippe dichtet nämlich nur relativ schlecht gegen den Spritzenzylinder ab. Dadurch ist es möglich, daß ein Teil des in dem Spritzenzylinder befindlichen Medikaments zwischen der ersten Dichtlippe und der Wand des Spritzenzylinders hindurch in den zwischen der ersten und der zweiten Dichtlippe befindlichen Rücksprung gelangt. Dieser Anteil des Medikaments verbleibt bei der Verabreichung des Medikaments ungenutzt zwischen den Lamellen, wodurch sich bei einer mit einer definierten Menge eines Medikaments vorgefüllten Spritze die applizierte Medikamentenmenge entsprechend reduziert. Darüber hinaus gerät der zwischen die Dichtlippen des Kolbenstopfens gelangende Anteil des Medikaments aber auch mit dem unbeschichteten Gummiwerkstoff des Kolbenstopfens in Kontakt, wodurch sich die Haltbarkeit des medikamentösen Spritzeninhaltes reduzieren kann.

Die Herstellung des vorbekannten Kolbenstopfens erfolgt in mehreren Arbeitsschritten. In einem ersten Arbeitsschritt wird zunächst zur Formung eines ersten, den von dem Inertfilm kappenförmig umschlossenen Kolbenabschnitt aufweisenden Kolbenstopfenanteils ein unvulkanisiertes Gummifeld zusammen mit einem Inertfilm in eine erste Hohlform einer Kaliberplatte eines Formwerkzeugs eingedrückt. Dabei wird das Gummifell unter Einwirkung von Druck und Wärme vulkanisiert und untrennbar mit dem Inertfilm verbunden. In einem weiteren Arbeitsschritt wird das so geformte Kolbenstopfenteil aus dem Formwerkzeug entnommen und aus dem beschichteten Gummifell ausgestanzt. Dabei bildet der Stanzrand bei dem fertig hergestellten Kolbenstopfen den Rücksprung zwischen der mit dem Inertfilm beschichteten ersten Dichtlippe und der dazu benachbarten zweiten Dichtlippe. Das so hergestellte erste Kolbenstopfenteil wird dann in einem weiteren Arbeitsschritt in eine Hohlform eines zweiten Formwerkzeugs eingelegt. In dieses zweite Formwerkzeug wird ein zweites, unvulkanisiertes Gummifell eingelegt, aus dem unter Einwirkung von Druck und Wärme ein unbeschichtetes zweites Kolbenstopfenteil geformt wird, das sich mit dem ersten Kolbenstopfenteil zu dem im wesentlichen fertigen Kolbenstopfen verbindet. Bei der Formung in dem zweiten Formwerkzeug wird in die dem Inertfilm gegenüberliegende Stirnseitenfläche des Kolbenstopfens eine mit Abstand von dem ersten Kolbenstopfenteil endende Aufnahmehöhlung zum Verbinden mit der Kolbenstange einer Spritze eingeformt. Danach wird der Kolbenstopfen aus dem zweiten Gummifell ausgestanzt.

Das vorbekannte Verfahren hat den Nachteil, daß für die Herstellung des Kolbenstopfens zwei Formungsschritte und zwei Stanzvorgänge erforderlich sind. Das Verfahren ist deshalb vergleichsweise aufwendig. Auch ist das Verfahren für eine Serienfertigung nur bedingt geeignet. In dem ersten Formwerkzeug können zwar eine Vielzahl von ersten Kolbenstopfenteilen gleichzeitig geformt werden, jedoch werden diese nach der Entnahme aus dem Formwerkzeug aus dem Gummifell ausgestanzt und vereinzelt. Das Einlegen der einzelnen Kolbenstopfenteile in ein zweites Formwerkzeug, das eine der Anzahl der mit dem ersten Formwerkzeug hergestellten Kolbenstopfenteilen entsprechende Anzahl von Formhöhlungen aufweist, ist deshalb vergleichsweise kompliziert.

Es besteht deshalb die Aufgabe, einen Kolbenstopfen der eingangs genannten Art zu schaffen, der einfach und kostengünstig herstellbar ist, der eine bessere Abdichtung eines Spritzen- oder Karpulenzylinders ermöglicht und bei dem eine Wechselwirkung zwischen dem Kolbenwerkstoff und einem in dem Spritzen- oder Karpulenzylinder befindlichen Inhalt zuverlässig vermieden wird. Außerdem besteht die Aufgabe, ein Verfahren und eine Vorrichtung der eingangs genannten Art anzugeben, die eine einfache und kostengünstige Herstellung eines solchen Kolbenstopfens ermöglichen.

Die Lösung dieser Aufgabe besteht bezüglich des Verfahrens darin, daß während des Formungsschritts aus der Schichtanordnung die bis auf einen im Bereich der Trennebene des Formwerkzeugs verbleibende, seitlich über den Kolbenstopfen überstehenden Schichtanordnungsflansch vollständige Kontur des Kolbenstopfens gebildet wird, daß die Aufnahmehöhlung während des Formungsschritts bis über den Schichtanordnungsflansch hinaus in den von dem Inertfilm kappenförmig umschlossenen Kolbenabschnitt in das Gummifell eingebracht wird, daß in einem Abtrennvorgang zumindest der die Aufnahmehöhlung umgenzende, den Schichtanordnungsflansch überragende Wandungsbereich des Dichtabschnitts in die Aufnahmehöhlung verdrängt und der Kolbenstopfen dann durch einen Stanzvorgang von dem Schichtanordnungsflansch abgetrennt wird, so daß in dem Dichtabschnitt eine ringförmig umlaufende, sich unmittelbar an Rand des den Kolbenabschnitt kappenförmig umschließenden Inertfilms anschließende Dichtzone gebildet wird, die in Gebrauchsstellung an dem Spritzen- oder Karpulenzylinder anliegt.

In vorteilhafter Weise ist es dadurch möglich, den Kolbenstopfen mit nur einem einzigen Formungsschritt und nur einem einzigen Stanz- beziehungsweise Abtrennvorgang herzustellen. In dem Formungsschritt wird die im wesentlichen vollständige Kontur des Kolbenstopfens aus der Schichtanordnung, bestehend aus dem Gummifell und dem Inertfilm, gebildet. Dabei wird die Aufnahmehöhlung an dem dem Inertfilm gegenüberliegenden Ende des Kolbenstopfens bis über die mit dem Inertfilm beschichtete Oberflächenebene des seitlich über den Kolbenstopfen überstehenden Schichtanordnungsflanschs hinaus in den von dem Inertfilm kappenförmig umschlossenen Kolbenabschnitt in das Gummifell eingebracht. Der die Aufnahmehöhlung umgrenzende Wandungsbereich des Dichtabschnitts kann deshalb beim Abstanzen des Schichtanordnungsflansches von dem Kolbenstopfens von dem Stanz- oder Schneidwerkzeug in die Aufnahmehöhlung verdrängt werden. Dadurch ist es möglich, den Schichtanordnungsflansch so von dem Kolbenstopfen abzutrennen, daß in dem Dichtabschnitt des Kolbenstopfens eine ringförmig umlaufende, sich unmittelbar an den Rand des den Kolbenabschnitt kappenförmig umschließenden Inertfilms anschließende Dichtzone gebildet wird, die mit der in Gebrauchsstellung an dem Spritzen- oder Karpulenzylinder anliegenden Randbereich des Inertfilms fluchtet oder radial etwas über diesen übersteht. Somit ist der Kolbenstopfen in Gebrauchsstellung unmittelbar im Anschluß an den Inertfilm durch die Dichtzone gegen die Wandung des Spritzen- oder Karpulenzylinders abgedichtet, wodurch ein Kontakt des Inhalts des Spritzen- oder Karpulenzylinders mit hinter der Dichtzone liegenden, unbeschichteten Bereichen des Kolbenstopfens zuverlässig vermieden wird.

Bei einer vorteilhaften Ausführungsform der Erfindung wird während des Formungsschritts wenigstens eine am Außenumfang des Dichtabschnitts ringförmig umlaufende Dichtlippe gebildet. Der nach dem Verfahren hergestellte Kolbenstopfen dichtet dann in Gebrauchsstellung noch besser gegen die Wand des Spritzen- oder Karpulenzylinders ab. Dadurch wird inbesondere das Eindringen von Gasen und/oder Keimen in den Inhalt des Spritzen- oder Karpulenzylinders grundsätzlich erschwert. Außerdem ermöglicht die Dichtlippe ein leichtgängiges Gleiten des Kolbenstopfens in dem Spritzen- oder Karpulenzylinder. Durch die bei dem Abtrennvorgang gebildete Dichtzone wird ausgeschlossen, daß der Inhalt des Spritzen- oder Karpulenzylinders in den Zwischenraum zwischen der Dichtlippe und dem von dem Inertfilm kappenförmig umschlossenen Kolbenabschnitt gelangt und dort mit dem elastomeren Kolbenwerkstoff in Wechselwirkung treten kann.

Bei einer bevorzugten Ausführungsform der Erfindung ist vorgesehen, daß in dem Abtrennschritt der wenigstens eine von dem Inertfilm kappenförmig umschlossene Kolbenabschnitt zentrisch in eine Öffnung einer Schneidmatrize eingesetzt und der den Kolbenstopfen umgrenzende Schichtanordnungsflansch zwischen dieser Schneidmatrize und einer Niederhalterplatte eingespannt wird, wobei der den Schichtanordnungsflansch überragende Teil des Dichtabschnitts in eine in der Niederhalterplatte vorgesehene Durchtrittsöffnung eingreift, und daß danach der die Aufnahmehöhlung des Kolbenstopfens umgenzende Wandungsbereich des Dichtabschnitts von einem Schneidstempel in die Aufnahmehöhlung verdrängt und der Schichtanordnungsflansch von dem Kolbenstopfen abgestanzt wird. Der Kolbenstopfen ist dann mit seinem in die Öffnung der Schneidmatrize eingesetzten Kolbenabschnitt in Bezug zu der Längsachse des Schneidwerkzeugs zentriert und wird während des Stanzvorgangs über den zwischen der Schneidmatrize und der Niederhalterplatte eingespannten Schichtanordnungsflansch in Zentrierlage fixiert. Zusätzlich ist der in die Durchtrittsöffnung der Niederhalterplatte eingreifende und an deren Wandung anliegende Teilbereich des Dichtabschnitts in der Durchtrittsöffnung relativ zu dem Schneidwerkzeug zentriert. Der die Aufnahmehöhlung umgrenzende Wandungsbereich des Dichtabschnitts kann dann bei der Stanzvorschubbewegung des Schneidwerkzeugs leichter von diesem in die Aufnahmehöhlung verdrängt werden.

Besonders vorteilhaft ist, wenn der Schneidstempel zum Abtrennen des Kolbenstopfens von dem Schichtanordnungsflansch von dem freien Ende des Dichtabschnitts her auf den Schichtanordnungsflansch zubewegt wird. Der die Aufnahmehöhlung umgrenzende Wandungsbereich des Kolbenstopfens kann dann von dem Schneidwerkzeug noch besser in die Aufnahmehöhlung verdrängt werden.

Bei einer besonders vorteilhaften Ausführungsform der Erfindung ist vorgesehen, daß das Gummifell an seinem Außenrand umlaufend gegen wenigstens eine daran anliegende Formplatte des Formwerkzeugs abgedichtet wird und daß während und/oder nach dem Öffnen des Formwerkzeugs zum Ablösen der vulkanisierten Schichtanordnung von der Formplatte zwischen dieser und der Schichtanordnung ein Gas eingeblasen wird. Die vulkanisierte Schichtanordnung wird dann vor dem Entnehmen aus der Formplatte gleichmäßig mit dem Druck des eingeblasenen Gases beaufschlagt, was ein schonendes Entformen der Schichtanordnung ermöglicht. Dabei wird eine Überdehnung des vulkanisierten Gummis und/oder des Inertfilms vermieden. Die nach dem Verfahren hergestellten Kolbenstopfen weisen deshalb eine hohe Maßhaltigkeit auf, wodurch insbesondere das Einlegen der vulkanisierten Schichtanordnung in die Schneidmatritze ermöglicht wird.

Vorteilhaft ist, wenn zum Abdichten der vulkanisierten Schichtanordnung gegen die Formplatte während des Formungsschritts am Außenrand der Schichtanordnung eine um die Schichtanordnung umlaufende, an der Formplatte dichtend anliegende Dichtung geformt wird. Die für die Abdichtung des Gummifells erforderliche Dichtung wird also zusammen mit dem (den) Kolbenstopfen in einem Arbeitsschritt hergestellt. Das Verfahren ist dadurch noch einfacher durchführbar.

Die vorstehend genannte Aufgabe wird bezüglich der Vorrichtung dadurch gelöst, daß das Formwerkzeug zur Formung der bis auf den im Bereich der Trennebene des Formwerkzeugs verbleibenden, seitlich über den Kolbenstopfen überstehenden Schichtanordnungsflansch vollständigen Kontur wenigstens eines Kolbenstopfens ausgebildet ist und daß dazu die erste Formplatte wenigstens eine erste, an den von dem Inertfilm kappenförmig umschlossenen Kolbenabschnitt des herzustellenden Kolbenstopfens formangepaßte Kavität und die zweite Formplatte wenigstens eine zweite, an den unbeschichteten Dichtabschnitt des Kolbenstopfens formangepaßte Kavität aufweist, daß in der zweiten Kavität ein Formkern zur Formung einer mit der Kolbenstange oder dergleichen Bewegungsübertragungselement verbindbaren Aufnahmehöhlung des Kolbenstopfens angeordnet ist, der in Schließstellung des Formwerkzeugs mit seinem freien Ende in die gegenüberliegende erste Kavität eingreift, daß die Stanzvorrichtung eine Schneidmatrize mit wenigstens einer Öffnung zum zentrischen Einsetzen des von dem Inertfilm kappenförmig umschlossenen Kolbenabschnitts hat, daß der Schneidmatrize zum Abtrennen des Schichtanordnungsflansches 16 von dem Kolbenstopfen ein hülsenförmiger, an seinem Außenumfang eine Schneidkante aufweisender Schneidstempel zugeordnet ist, der axial zur Öffnung der Schneidmatrize auf diese zu- und von dieser wegbewegbar ist, und daß die Innenhöhlung des Schneidstempels zur Aufnahme des nach innen verformten Dichtabschnitts des Kolbenstopfens ausgebildet ist.

In vorteilhafter Weise ermöglicht es die Vorrichtung, den Kolbenstopfen mit nur je einem einzigen Formungsschritt und nur je einem einzigen Stanzvorgang herzustellen. Da der in der Kavität der zweiten, in Gebrauchsstellung an dem Gummifell anliegenden Formplatte vorstehende Formkern zur Formung der mit einer Kolbenstange oder dergleichen Bewegungsübertragungselement verbindbaren Aufnahmehöhlung des Kolbenstopfens in Schließstellung des Formwerkzeugs mit seinem freien Ende in die gegenüberliegende Kavität der ersten, in Gebrauchsstellung an dem Inertfilm anliegenden Formplatte eingreift, wird die Aufnahmehöhlung beim Formen des Kolbenstopfens in dem Formwerkzeug bis in den von dem Inertfilm kappenförmig umschlossenen Kolbenabschnitt eingebracht. Die vulkanisierte Schichtanordnung, bestehend aus dem Gummifell und dem Inertfilm, wird dann nach der Entnahme aus dem Formwerkzeug mit dem von dem Inertfilm kappenförmig umschlossenen Kolbenabschnitt zentrisch in die Öffnung der Schneidmatrize eingesetzt. Bei der Vorschubbewegung des axial zu dieser Öffnung verschiebbaren, hülsenförmigen Schneidstempels wird der die Aufnahmehöhlung seitlich umgrenzende Wandungsbereich des Kolbens von dem Schneidstempel quer zur Vorschubbewegung nach innen ausgelenkt und in die Innenhöhlung des Schneidstempels verdrängt. Dadurch ist es möglich, den Schneidstempel auf das freie Ende des Dichtabschnitts des Kolbenstopfens aufzuschieben, ohne dieses zu beschädigen. Im weiteren Verlauf der Stanzvorschubbewegung des Schneidstempels durchdringt der Schneidstempel den seitlich über den Kolbenstopfen überstehenden Schichtanordnungsflansch und trennt diesen von den Kolbenstopfen ab. Dabei wird eine ringförmige, um den Dichtabschnitt umlaufende Dichtzone gebildet, die in Gebrauchsstellung an dem Spritzen- oder Karpulenzylinder anliegt. Die Außenquerschnittsabmessungen des an seinem Außenumfang die Schneidkante aufweisenden Schneidstempels entsprechen dann den Querschnittsabmessungen der Öffnung der Schneidmatrix oder sind etwas kleiner als diese. Die Querschnittsabmessungen der zur Formung des kappenförmig umschlossenen Kolbenabschnitts ausgebildeten Kavität der ersten Formplatte und somit auch die Querschnittsabmessungen dieses Kolbenabschnitts entsprechen etwa denjenigen der Öffnung der Schneidmatrix. Bei dem fertig hergestellten Kolbenstopfen fluchtet deshalb die um den Dichtabschnitt umlaufende, unmittelbar an den Inertfilm angrenzende Dichtzone mit dem in Gebrauchsstellung an dem Spritzen- oder Karpulenzylinder anliegenden Randbereich des Inertfilms oder steht radial etwas über diesen über, wodurch der Dichtabschnitt zuverlässig gegen den Spritzen- oder Karpulenzylinder abdichtet.

Die Formplatten des Formwerkzeugs können zum gleichzeitigen Formen einer Vielzahl von Kolbenstopfen jeweils eine Anzahl der zu formenden Kolbenstopfen entsprechende Anzahl von Kavitäten aufweisen. Die Schneidmatrize der Stanzvorrichtung kann dann eine der Anzahl der Kavitäten einer Formplatte entsprechende Anzahl Öffnungen mit jeweils einem daran angeordneten Schneidstempel aufweisen, wobei die Anordnung der Öffnungen derjenigen der Kavitäten der Formplatte entspricht, so daß die vulkanisierte Schichtanordnung zum gleichzeitigen Ausstanzen sämtlicher daran angeordneter Kolbenstopfen mit den kappenförmig umschlossenen Kolbenabschnitten in die Schneidmatrize eingelegt werden kann.

Bei einer vorteilhaften Ausführungsform der erfindungsgemäßen Vorrichtung ist vorgesehen, daß die Stanzvorrichtung eine Niederhalterplatte mit zumindest einer zu der wenigstens einen Öffnung der Schneidmatrize fluchtenden Durchtrittsöffnung zur Aufnahme des Dichtabschnitts hat, daß die Niederhalterplatte zum Einspannen des Schichtanordnungsflansches zwischen ihr und der Schneidmatrize axial zur Öffnung der Schneidmatrize relativ zu der Schneidmatrize verschiebbar ist, und daß der Schneidstempel in der Durchtrittsöffnung der Niederhalterplatte axial verschiebbar geführt ist. Der Schichtanordnungsflansch ist dann zwischen der Schneidmatrize und der Niederhalterplatte einspannbar, so daß der daran angeordnete Kolbenstopfen beim Stanzvorgang in Zentrierlage fixiert ist. Zusätzlich ist das freie Ende des Dichtabschnitts in der Durchtrittsöffnung der Niederhalterplatte in bezug zu dem hülsenförmigen Schneidstempel zentriert.

Bei einer bevorzugten Ausführungsform der Erfindung weist die Umfangswand der wenigstens einen Kavität der zweiten Formplatte zumindest eine ringförmig umlaufende Vertiefung zur Bildung einer am Außenumfang des Kolbenstopfen-Dichtabschnitts ringförmig umlaufenden Dichtlippe auf. Die mit dem Formwerkzeug hergestellten Kolbenstopfen dichten dann noch besser gegen den Spritzen- oder Karpulenzylinder ab.

Zweckmäßigerweise weist der Schneidstempel an seinen der Schneidmatrize zugewandten freien Endbereich innenseitig eine Einführschräge auf. Die die Aufnahmehöhlung umgrenzende Wandung des Kolbenstopfens kann dann bei der Stanzvorschubbewegung des hülsenförmigen Schneidstempels von diesem leichter in die Innenhöhlung des Schneidstempels verdrängt werden.

Bei einer besonders vorteilhaften Ausführungsform der Erfindung ist vorgesehen, daß der Außenrand der vulkanisierten Schichtanordnung mittels einer an der Schichtanordnung umlaufenden Dichtung gegen wenigstens eine Formplatte abdichtbar ist, und daß in dem von der Dichtung umgrenzten Bereich dieser Formplatte ein Gaszuführkanal mündet, der zum Ablösen der Schichtanordnung von der Formplatte mit einer Gasdruckquelle verbindbar ist. Die vulkanisierte Schichtanordnung mit dem (den) daran angeformten Spritzkolben kann dann durch den Gasdruck auf einfache Weise aus der zweiten Formplatte entformt werden. Dabei ermöglicht der Gasdruck eine gleichmäßige und schonende Ablösung der Schichtanordnung von der Formplatte. Dies ist insbesondere dann von Vorteil, wenn die Kavität(en) der Formplatte Rücksprünge aufweist (aufweisen), welche die vulkanisierte Schichtanordnung hintergreifen.

Vorteilhaft ist, wenn wenigstens eine Formplatte zur Bildung der den Außenrand der Schichtanordnung gegen die Formplatte abdichtenden Dichtung innerhalb ihrer Formhöhlung eine die Kavität(en) dieser Formplatte mit Abstand umgrenzende Ringnut aufweist. Die Dichtung wird dann während des Formens des Kolbenstopfens (der Kolbenstopfen) an den Rand der Schichtanordnung angeformt, vorzugsweise an das Gummifell.

Zweckmäßigerweise ist in den Mündungsbereich des Gaszuführkanals ein Ventil angeordnet, vorzugsweise ein Tellerventil. Der Gaszuführkanal ist dann während des Formens der Kolbenstopfen durch das Ventil abgedichtet, so daß der elastomere Werkstoff und/oder der Inertfilm nicht in den Gaszuführkanal eindringen können.

Die vorstehend genannte Aufgabe wird bezüglich des pharmazeutischen Kolbenstopfens dadurch gelöst, daß der Grundkörper einstückig ausgebildet ist, daß sich die Aufnahmehöhlung bis in den von dem Inertfilm kappenförmig umschlossenen Kolbenabschnitt erstreckt, und daß der Dichtabschnitt an seinem Außenumfang eine ringförmig umlaufende, sich unmittelbar an den Rand des den Kolbenabschnitt kappenförmig umschließenden Inertfilms anschließende Dichtzone aufweist, die in Gebrauchsstellung an dem Spritzen- oder Karpulenzylinder flächig anliegt.

Somit ist der Kolbenstopfen in Gebrauchsstellung unmittelbar im Anschluß an den Inertfilm durch die Dichtzone gegen die Wandung des Spritzen- oder Karpulenzylinders abgedichtet, wodurch ein Kontakt des Inhaltes des Spritzen- oder Karpulenzylinders mit hinter der Dichtzone liegenden, unbeschichteten Bereichen des Kolbenstopfens zuverlässig vermieden wird. Da der Grundkörper einstückig ausgebildet ist, ist der Kolbenstopfen einfach und kostengünstig aus einem einzigen, mit einem Inertfilm beschichteten Gummifell herstellbar.

Vorteilhaft ist, wenn die Dichtzone in gerader Verlängerung zu dem in Gebrauchsstellung an dem Spritzen- oder Karpulenzylinder anliegenden Außenumfangsabschnitt des Inertfilms angeordnet ist oder radial etwas über diesen Außenumfangsabschnitt übersteht. Die Dichtzone dichtet dann in Gebrauchsstellung unmittelbar im Anschluß an den Inertfilm flächig gegen die Wandung des Spritzen- oder Karpulenzylinders ab.

Bei einer zweckmäßigen Ausgestaltung der Erfindung weist ein Abschnitt der die Aufnahmehöhlung umgrenzenden Innenwand des Kolbenstopfens ein Innengewinde zum Verbinden mit dem Gewinde der Kolbenstange oder dergleichen Bewegungsübertragungselement auf, wobei dieser Innengewinde-Abschnitt mit Abstand vom Boden der Aufnahmehöhlung endet. Der Kolbenstopfen ist dann formschlüssig mit der Kolbenstange oder dem Bewegungsübertragungselement verbindbar bzw. verschraubbar. Bei der Herstellung kann der Kolbenstopfen aus einer Schichtanordnung, bestehend aus einem unvulkanisierten Gummifell und einem fluorierten Polymerfilm oder dergleichen Inertfilm in einem Formwerkzeug geformt werden und ist danach gut aus dem Formwerkzeug entformbar.

Bei einer vorteilhaften Ausführungsform der Erfindung ist vorgesehen, daß der Innengewinde-Abschnitt des Kolbenstopfens an dem kappenförmig umschlossenen Kolbenabschnitt endet oder von diesem beabstandet ist, und daß sich an den Innengewinde-Abschnitt vorzugsweise ein zylindrischer oder konischer Aufnahmehöhlungsabschnitt anschließt, der sich bis in den kappenförmig umschlossenen Kolbenabschnitt erstreckt. Der Kolbenstopfen ist dann bei seiner Herstellung noch leichter aus dem Formwerkzeug entformbar.

Bei einer anderen Ausfführungsform der Erfindung ist vorgesehen, daß sich der Querschnitt der Aufnahmehöhlung ausgehend vom Boden zur Öffnung der Aufnahmehöhlung hin konisch erweitert. Auch dieser, zum Verbinden mit der Kolbenstange einer Karpule vorgesehene Kolbenstopfen ist bei seiner Herstellung noch leichter aus dem Formwerkzeug entformbar.

Nachfolgend sind Ausführungsbeispiele der Erfindung anhand der Zeichnung näher erläutert. Es zeigen:
- Fig. 1: einen Querschnitt durch ein in Offenstellung befindliches Formwerkzeug, in das ein einseitig mit einem Inertfilm beschichtetes Gummifell eingelegt ist,
- Fig. 2: eine Darstellung ähnlich Fig. 1, wobei jedoch das Formwerkzeug geschlossen ist,
- Fig. 3: eine im Querschnitt durch das Formwerkzeug während des luftunterstützten Entformens der aus dem vulkanisierten Gummifell und dem Inertfilm bestehenden Schichtanordnung,
- Fig. 4: einen Querschnitt durch das Formwerkzeug bei der Entnahme der vulkanisierten Schichtanordnung,
- Fig. 5 bis 8: einen Teilquerschnitt durch eine Stanzvorrichtung zum Abtrennen eines seitlich über einen Kolbenstopfen überstehenden, nur teilweise dargestellten Schichtanordnungsflanschs von dem Kolbenstopfen, wobei die Niederhalterplatte und der Stanzstempel der Stanzvorrichtung in unterschiedlichen Arbeitslagen dargestellt sind,
- Fig. 9 und 11: eine teilweise im Schnitt dargestellte Seitenansicht eines pharmazeutischen Kolbensstopfens,
- Fig. 10: eine teilweise im Schnitt dargestellte Seitenansicht einer Spritze und
- Fig. 11: eine teilweise im Schnitt dargestellte Seitenansicht einer Karpule.

Eine pharmazeutische Spritze oder Karpule weist einen Spritzenoder Karpulenzylinder 1 mit einem darin axial verschiebbar angeordneten Kolbenstopfen 2 auf. Der Spritzen- oder Karpulenzylinder 1 hat an seinem einen Ende eine mit einer entfernbaren Abdeckung 3 dicht verschlossene Austrittsöffnung für eine in der Zylinderhöhlung 4 des Spritzen- oder Karpulenzylinders 1 befindliche, flüssige pharmazeutische Zubereitung. An seinem der Austrittsöffnung gegenüberliegenden Ende weist der Spritzen- oder Karpulenzylinder 1 eine Durchtrittsöffnung für eine mit dem Kolbenstopfen 2 verbundene Kolbenstange 5 auf, die mit ihrem diesem zugewandten Ende in eine Aufnahmehöhlung 6 des Kolbenstopfens 2 eingreift.

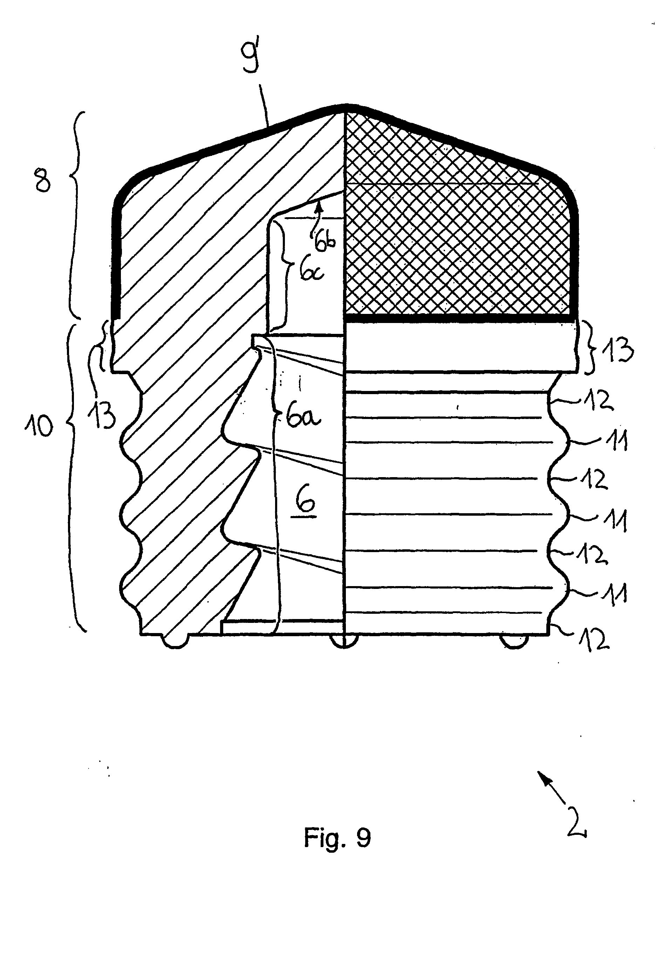

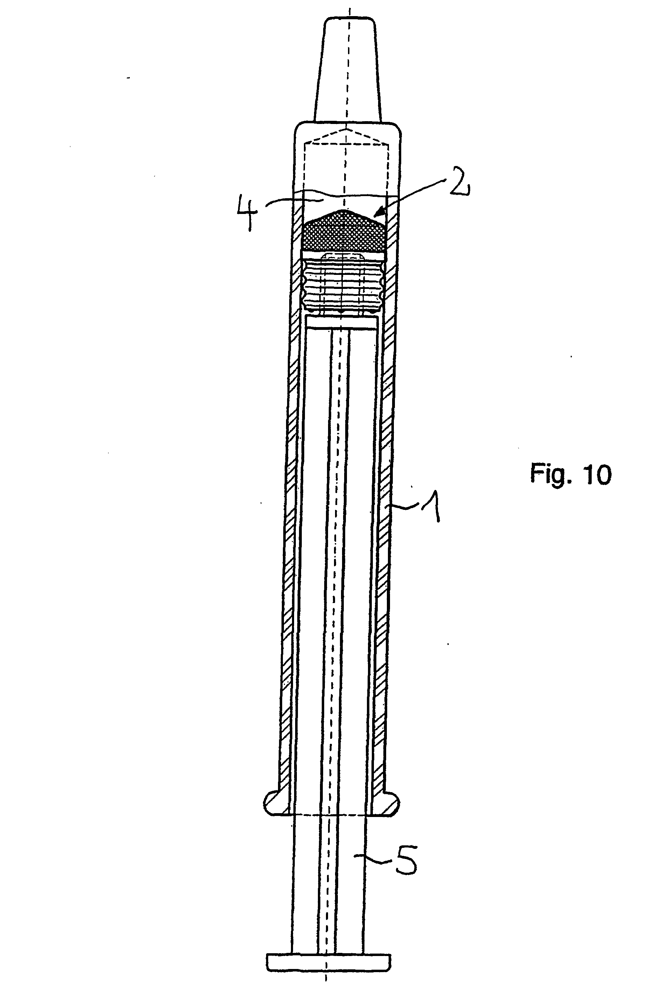

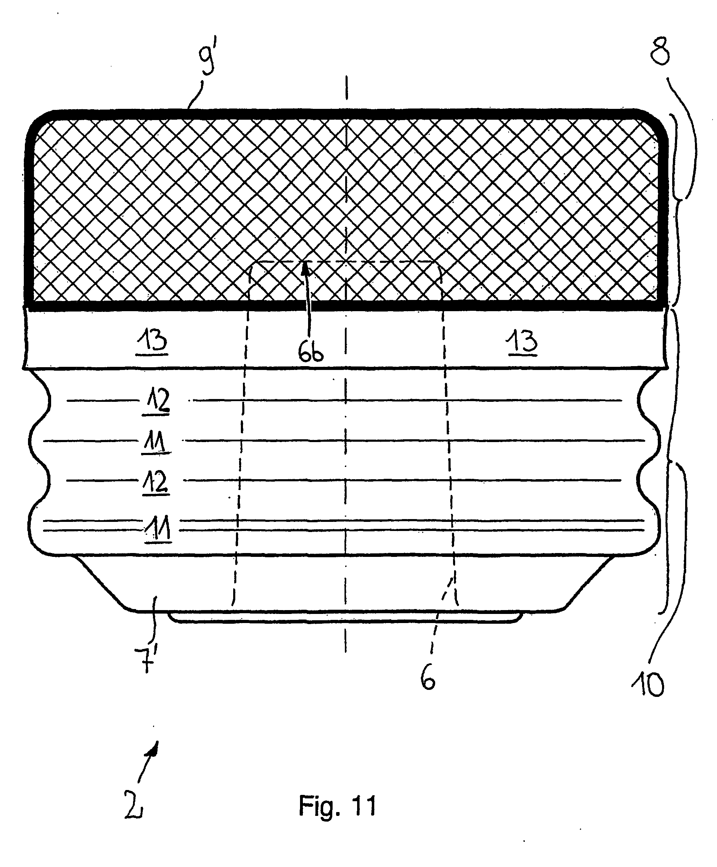

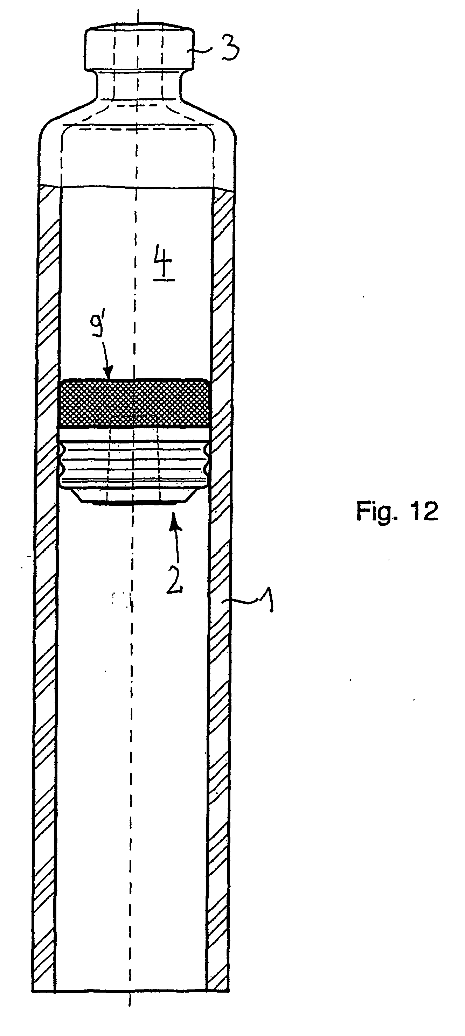

Wie in Fig. 9 und 11 besonders gut erkennbar ist, weist der Kolbenstopfen 2 einen einstückig ausgebildeten Grundkörper 7' aus Gummi oder dergleichen elastomerem Werkstoff auf, der an einem dem Inhalt des Spritzen- oder Karpulenzylinders 1 zugewandten Kolbenabschnitt 8 von einem fluorierten Polymerfilm oder dergleichen Inertfilm 9' kappenförmig umschlossen ist. An diesen Kolbenabschnitt 8 schließt sich ein unbeschichteter Dichtabschnitt 10 des Kolbenstopfens 2 an. In Fig. 10 und 12 ist erkennbar, daß der Kolbenstopfen 2 an seinem Außenumfang sowohl mit dem Randbereich des Inertfilms 9' als auch mit dem Dichtabschnitt 10 an der Innenwand des Karpulenzylinders 1 anliegt.

Der Dichtabschnitt 10 weist an seinem Außenumfang mehrere umlaufende Dichtlippen 11 auf, die durch dazwischen befindliche, am Außenumfang des Dichtabschnitts 10 umlaufende Rücksprünge 12 voneinander beabstandet sind. Auch zwischen der zu dem Inertfilm 9' benachbarten Dichtlippe 11 und dem Inertfilm 9' ist ein Rücksprung 12 vorgesehen. Zwischen diesem Rücksprung 12 und dem Inertfilm 9' weist der Dichtabschnitt 10 eine an seinem Außenumfang ringförmig umlaufende Dichtzone 13 auf, die sich unmittelbar an den Rand des den Kolbenabschnitt 8 kappenförmig umschließenden Inertfilms 9' anschließt. Die Dichtzone 13 fluchtet mit dem an der Innenwand des Spritzenoder Karpulenzylinders 1 anliegenden Oberfläche des Randbereichs des Inertfilms 9' oder steht radial etwas über diese Oberfläche über. Mit der Dichtzone 13 liegt der Kolbenstopfen 2 in Gebrauchsstellung flächig an dem Spritzenoder Karpulenzylinder 1 an. Dadurch sind die Rücksprünge 12 zuverlässig gegen die in der Zylinderhöhlung 4 befindliche flüssige pharmazeutische Zubereitung abgedichtet, so daß die Zubereitung dort nicht mit dem elastomeren Werkstoff in Wechselwirkung treten kann. Die Dichtzone 13 verhindert also, daß die pharmazeutische Zubereitung zwischen dem im Vergleich zu dem unbeschichteten elastomeren Werkstoff schlechter abdichtenden Inertfilm 9' und der Innenwand des Spritzen- oder Karpulenzylinders 1 hindurch in den benachbarten Rücksprung 12 eindringen kann. Darüber hinaus dichtet die Dichtzone 13 aber auch die pharmazeutische Zubereitung gegen das Eindringen von Feuchtigkeit, Gasen und/oder Keimen ab. Zusätzlich wird die Zylinderhöhlung 4 durch die mit der Dichtzone 13 in Reihe geschalteten Dichtlippen 11 abgedichtet.

Wie in Fig. 9 und 11 erkennbar ist, erstreckt sich die Aufnahmehöhlung 6 von ihrer an dem von dem Inertfilm 9' entfernten Endbereich des Kolbenstopfens 2 befindlichen Aufnahmehöhlung bis in dem von dem Inertfilm 9' kappenförmig umschlossenen Kolbenabschnitt 8 und endet dort mit Abstand zu dem Inertfilm 9'.

Bei dem Ausführungsbeispiel nach Fig. 9 weist ein Abschnitt 6a der Aufnahmehöhlung 6 ein Innengewinde auf, das mit einem dazu passenden Außengewinde der Kolbenstange 5 verschraubbar ist. Der Innengewinde-Abschnitt 6a des Kolbenstopfens 2 erstreckt sich innerhalb des von dem unbeschichteten Dichtabschnitt 10 umgrenzten Kolbenbereichs und endet mit Abstand zu dem kappenförmig umschlossenen Kolbenabschnitt 8. An den Innengewinde-Abschnitt 6a schließt sich ein etwa zylindrischer Aufnahmehöhlungsabschnitt 6c an, der sich bis in den kappenförmig umschlossenen Kolbenabschnitt 8 erstreckt. Es sind aber andere formschlüssige Verbindungen zwischen dem Kolbenstopfen 2 und der Kolbenstange 5 oder dergleichen Bewegungsübertragungselement denkbar. Bei dem Ausführungsbeispiel nach Fig. 11 ist die Aufnahmehöhlung 6 exemplarisch konusförmig ausgebildet und verjüngt sich ausgehend von ihrer Aufnahmeöffnung zur tiefsten Stelle der Aufnahmehöhlung 6 hin.

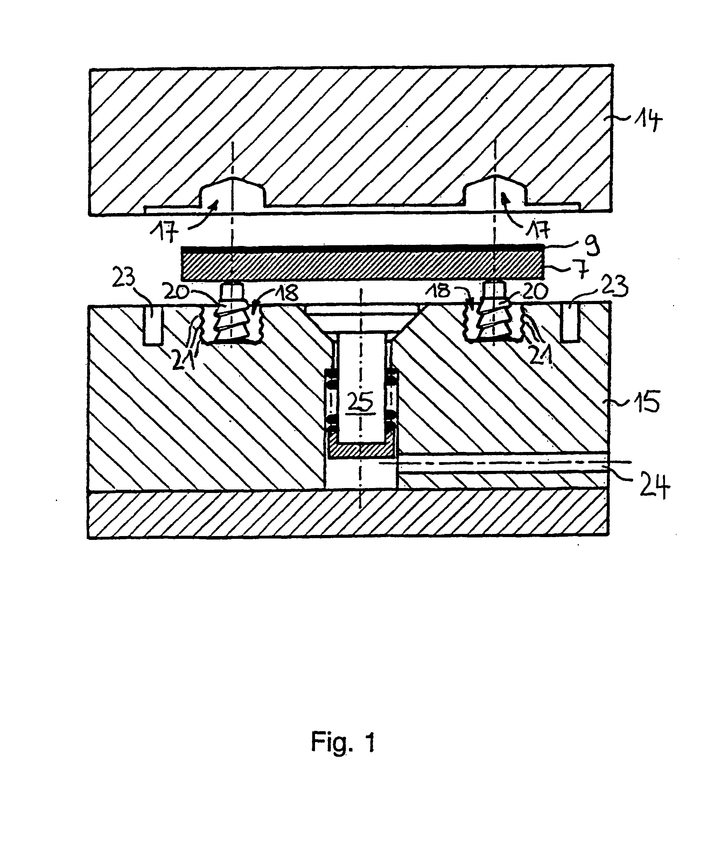

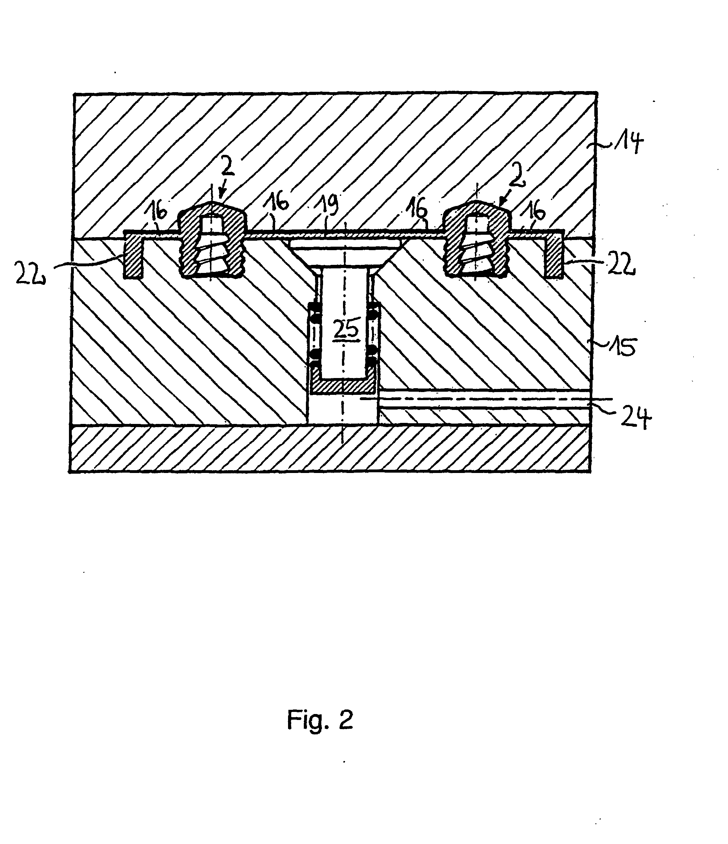

Zur Herstellung des Kolbenstopfens 2 wird zunächst ein unvulkanisiertes Gummifell 7 zusammen mit einem fluorierten Polymerfilm oder dergleichen Inertfilm 9 zwischen die Formplatten 14, 15 eines Formwerkzeugs eingelegt. Die Formplatten 14, 15 sind relativ zueinander bewegbar und in eine Schließstellung (Fig. 2) und eine Offenstellung (Fig. 1) bringbar. Der zunächst noch ebene, folienartige Inertfilm 9 kann - wie dies in Figur 1 gezeigt ist - mit dem Gummifell 9 fest verbunden sein. Der Inertfilm 9 und das Gummifell 9 können aber auch unabhängig voneinander zwischen die Formplatten 14, 15 eingebracht und lose aufeinander gelegt werden.

Das Formwerkzeug ist zur Formung bis auf den im Bereich der Trennebene des Formwerkzeugs verbleibenden, seitlich über den Kolbenstopfen 2 überstehenden Schichtanordnungsflansch 16 vollständigen Kontur des Kolbenstopfens 2 ausgebildet. In Fig. 1 ist erkennbar, daß die dem Inertfilm 9 zugewandte erste Formplatte 14 dazu mehrere erste Kavitäten 17 aufweist, deren Form der Negativform des von dem Inertfilm kappenförmig umschlossenen Kolbenabschnitts 8 des herzustellenden Spritzkolbens 2 entspricht. Die dem Gummifell 7 zugewandte zweite Formplatte 15 weist eine der Anzahl der ersten Kavitäten 17 entsprechende Anzahl zweiter Kavitäten 18 auf, deren Form der Negativform des sich an die Dichtzone anschließenden Teiles des Spritzkolben-Dichtabschnitts 10 entspricht. Die ersten und zweiten Kavitäten 17, 18 fluchten zueinander, so daß sie in Schließstellung des Formwerkzeuges jeweils im wesentlichen die Außenkontur des herzustellenden Spritzkolbens 2 umgrenzen. In Schließstellung des Formwerkzeuges sind die ersten und zweiten Kavitäten 17, 18 durch einen Trennspalt 19 voneinander beabstandet, der sich in der Trennebene der Formplatten 14, 15 über sämtliche Kavitäten 17, 18 des Formwerkzeugs erstreckt und diese miteinander verbindet. In dem Trennspalt 19 bildet sich beim Formen der aus dem Gummifell 7 und dem Inertfilm 9 bestehenden Schichtanordnung der Schichtanordnungsflansch 16 aus.

Wie in Fig. 2 besonders gut erkennbar ist, umgrenzenden die zweiten Kavitäten jeweils einen Formkern 20, zur Formung der mit der Kolbenstange 5 verbindbaren Aufnahmehöhlung 6 des Kolbenstopfens 2. Dieser Formkern 20 greift in Schließstellung des Formwerkzeugs mit seinem freien Ende in die gegenüberliegende erste Kavität 17 ein, so daß die Aufnahmehöhlung 6 beim Formen des Kolbenstopfens 2 bis in den von dem Inertfilm 9 kappenförmig umschlossenen Kolbenabschnitt 8 des Kolbenstopfens 2 eingebracht wird. Die Umfangswände der zweiten Kavitäten 18 weisen ringförmig umlaufende Vertiefungen 21 zur Bildung der am Außenumfang des Kolbenstopfen-Dichtabschnitts 10 ringförmig umlaufenden Dichtlippen 11 auf.

Beim Schließen des Formwerkzeugs wird das Gummifell 7 unter Einwirkung von Druck und Wärme vulkanisiert und verbindet sich untrennbar mit dem Inertfilm 9. Dabei nehmen die an den Wänden der ersten Kavitäten 17 anliegenden Abschnitte des Inertfilms 9 jeweils die Form der ersten Kavität 17 und die an der an den Wänden der zweiten Kavität 18 anliegenden Abschnitte des Gummifells 7 jeweils die Form der zweiten Kavitäten an.

Die zweite Formplatte weist zur Bildung einer den Außenrand der vulkanisierten Schichtanordnung gegen die Formplatte 14 abdichtenden Dichtung 22 innerhalb ihrer Formhöhlung eine die Kavitäten 18 dieser Formplatte 14 mit Abstand umgrenzende, umlaufende Ringnut 23 auf. In dieser Ringnut 23 wird beim Schließen des Formwerkzeugs das am Rand des Gummifells 7 befindliche elastomere Material verdrängt, wobei es die Form der Ringnut 23 annimmt.

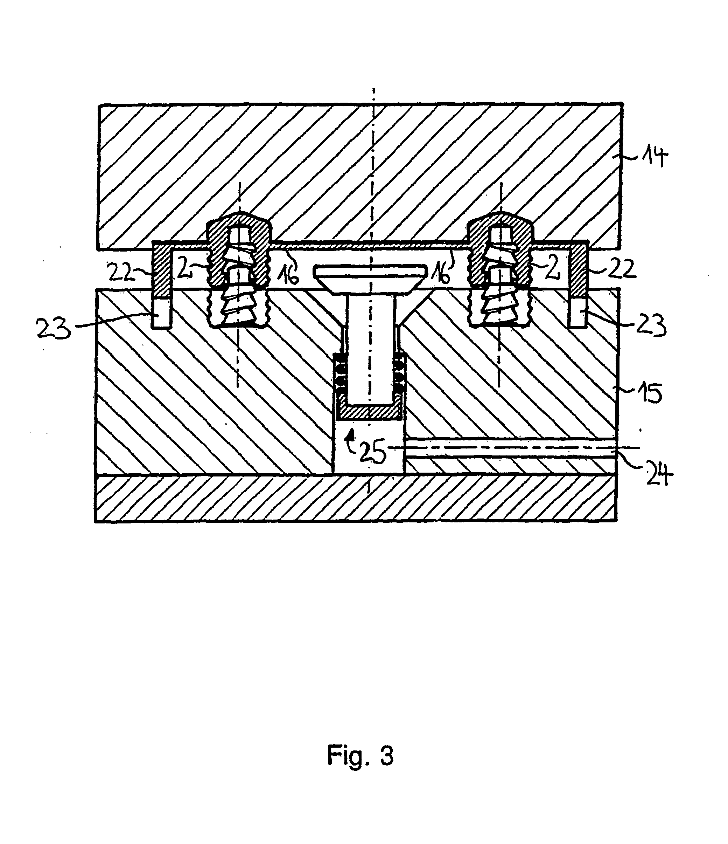

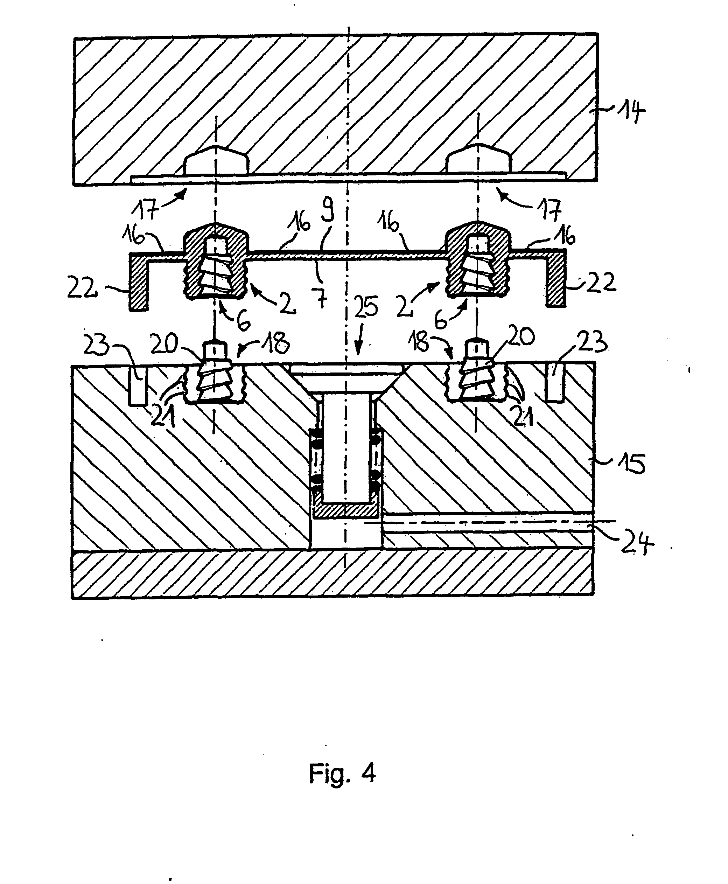

Die zweite Formplatte 14 hat einen Gaszuführkanal 24, der innerhalb des von der Ringnut 23 umgrenzten Bereichs in die zwischen den Formplatten 14, 15 gebildete Formhöhlung mündet. Durch diesen Gaszuführkanal 24 hindurch wird nach Beendigung des Formungsprozesses Druckluft eingeblasen, welche die aus dem vulkanisierten Gummifell 7 und dem Inertfilm 9 bestehende Schichtanordnung von der zweiten Formplatte 15 abhebt. In Figur 3 ist erkennbar, daß bei dem druckluftunterstützten Entfernen der Schichtanordnung von der zweiten Formplatte die Dichtung zunächst mit der Ringnut 23 in Eingriff bleibt, so daß sich zwischen dem Gummifell 7 und der zweiten Formplatte 15 ein Luftpolster ausbilden kann. Gleichzeitig wird das Formwerkzeug geöffnet, so daß die Schichtanordnung während des Öffnens zunächst noch mit ihrem Inertfilm 9 an der ersten Formplatte 14 anliegend bleibt, wie dies in Figur 3 erkennbar ist. Es ist aber auch eine andere Vorgehensweise möglich, bei der zunächst das Formwerkzeug geöffnet und mit der ersten Formplatte 14 von der Schichtanordnung entfernt wird und danach die Druckluft zwischen die Schichtanordnung und die zweite Formplatte eingeblasen wird. Nach dem Ablösen der Schichtanordnung aus der zweiten Formplatte 15 wird die Schichtanordnung aus dem Formwerkzeug entnommen. Dabei löst sich der Inertfilm 9 von der ersten Formplatte 14 ab (Fig. 4).

Wie in Figur 3 besonders gut erkennbar ist, ist in dem Mündungsbereich des Gaszuführkanals 24 ein Ventil 25 angeordnet, das mit seinem Ventilteller der ersten Formplatte 14 zugewandt ist. Deutlich ist erkennbar, daß das Ventil außerhalb der Kavitäten 18 angeordnet ist und seitlich von diesen beabstandet ist. Der Ventilteller des Ventils 25 wird durch eine Schraubenfeder in Schließstellung gehalten und ist durch den Druck der durch den Gaszuführkanal 24 zuführbaren Druckluft von seinem Ventilsitz entgegen der Rückstellkraft der Schraubenfeder abhebbar.

Nach der Entnahme aus dem Formwerkzeug wird die Schichtanordnung an einer Stanzvorrichtung 26 positioniert, um die Kolbenstopfen 2 vom dem sie umgrenzenden Schichtanordnungsflansch 16 abzutrennen. Die Stanzvorrichtung 26 weist eine Schneidmatrize 27 auf, die als Platte mit einer der Anzahl der ersten Kavitäten 14 entsprechenden Anzahlöffnungen 28 ausgebildet ist. Der Durchmesser dieser Öffnungen 28 entspricht etwa dem Durchmesser der ersten Kavitäten 14. Auch die relative Lage der Öffnungen 28 zueinander entspricht der relativen Lage, welche die Kavitäten 14 zueinander aufweisen. Die Schichtanordnung wird so an der Stanzmatrize 27 angeordnet, so daß die einzelnen, von dem Inertfilm 9 kappenförmig umschlossenen Kolbenabschnitte 8 der Schichtanordnung jeweils zentrisch in die ihnen zugeordnete Öffnung 28 eingreifen und der Schichtanordnungsflansch 16 auf der Schneidmatrize 27 aufliegt.

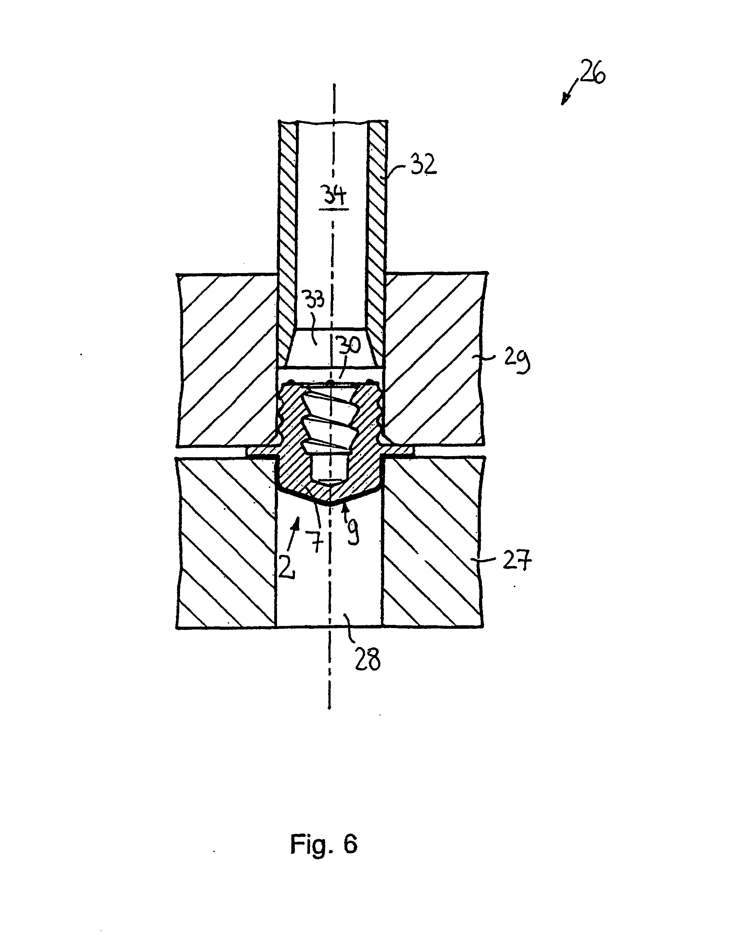

Parallel zu der Ebene der Schneidmatrize 27 ist eine Niederhalterplatte 29 angeordnet, die eine der Anzahl der Öffnungen 28 der Schneidmatrize 27 entsprechende Anzahl Durchtrittsöffnungen 30 hat, die den gleichen Durchmesser aufweisen, wie die Öffnungen 28 der Schneidmatrize 27 und mit den Öffnungen 28 in gerader Verlängerung fluchten. Nach dem Einlegen der Schichtanordnung in die Stanzmatrize 27 wird die Niederhalterplatte 29 auf die Stanzmatrize 27 zubewegt, bis der Schichtanordnungsflansch zwischen der Niederhalterplatte 29 und der Stanzmatrize 27 eingespannt und die daran angeordneten Kolbenstopfen 2 somit an der Stanzmatrize 27 fixiert sind (Fig. 6).

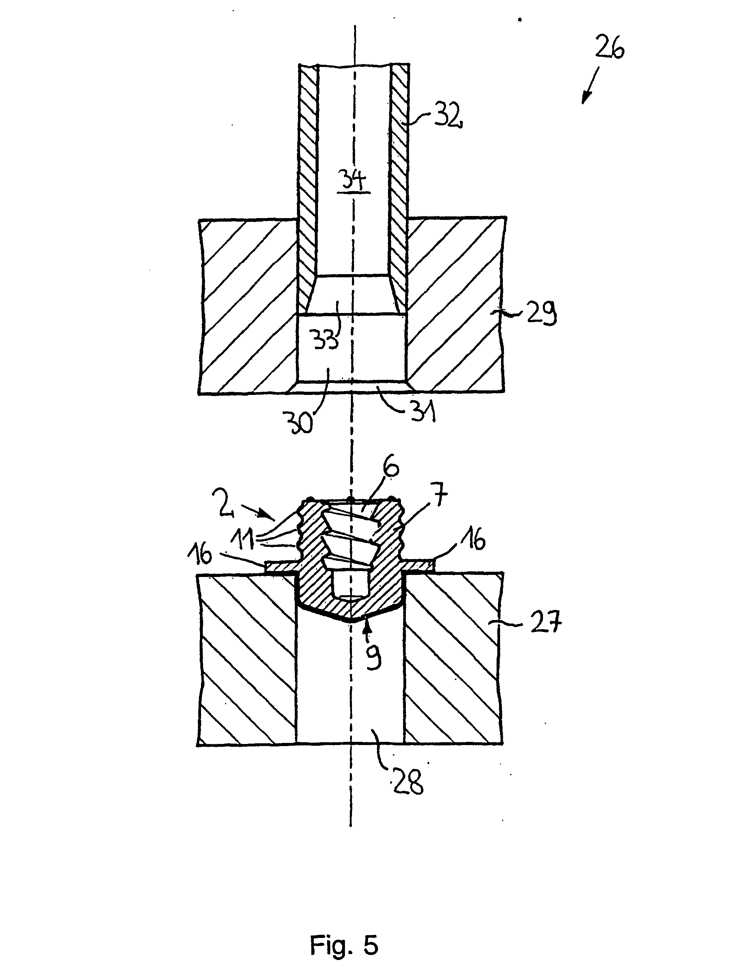

In Figur 5 ist erkennbar, daß an dem der Stanzmatrize 27 zugewandten Endbereich der Durchtrittsöffnungen 30 jeweils eine durch eine Anphasung gebildete Einführschräge 31 für den in die Durchtrittsöffnung 30 einzuführenden Dichtabschnitt 10 des Kolbenstopfens 2 vorgesehen ist. An dieser Einführschräge 31 wird der Dichtabschnitt 10 des Kolbenstopfens 2 zentriert, wenn die Niederhalterplatte 29 auf die Stanzmatrize 26 zubewegt wird.

In den einzelnen Durchtrittsöffnungen 30 ist jeweils ein hülsenförmiger Schneidstempel 32 axial verschiebbar geführt, der an seinem Außenumfang eine Schneidkante aufweist. In den Figuren 6 bis 8 ist erkennbar, daß die Schneidstempel 32 zum Abtrennen des Schichtanordnungsflansches 16 von dem an den Schneidstempel 32 jeweils positionierten Kolbenstopfen auf die Schneidmatrize 27 zubewegt und nach dem Abtrennen des Schichtanordnungsflansches 16 wieder in ihre Ausgangslage zurückgezogen werden.

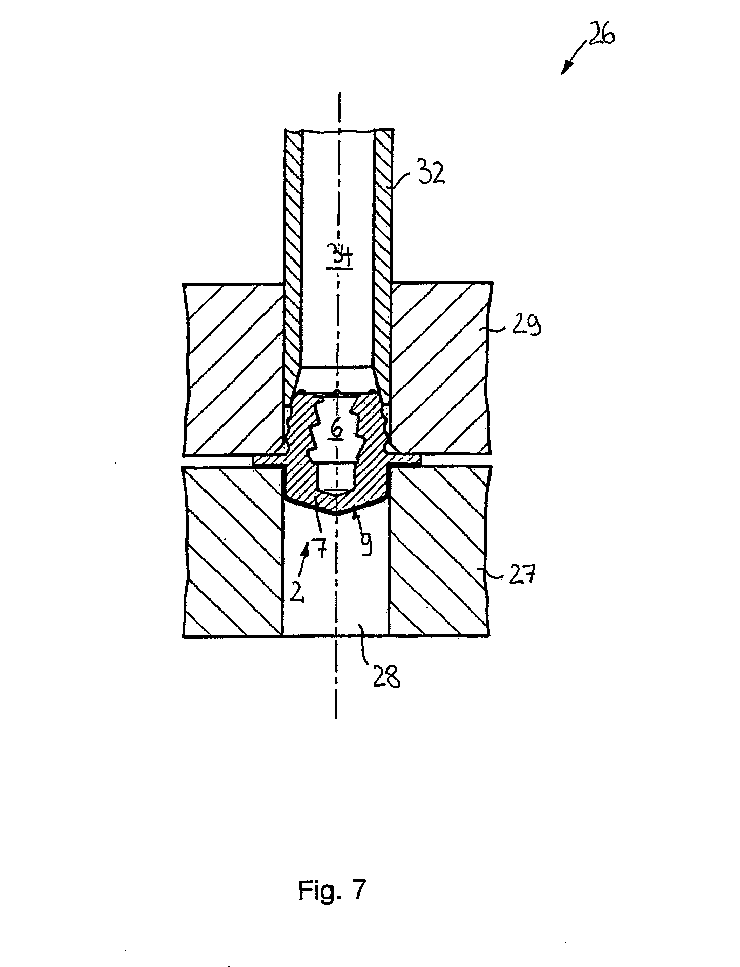

Die Schneidstempel 32 weisen jeweils an ihrem der Schneidmatrize 27 zugewandten freien Endbereich innenseitig eine Einführschräge 33 auf. In Figur 7 ist erkennbar, daß bei der Schneidvorschubbewegung des Schneidstempels 32 an dieser Einführschräge 33 die die Aufnahmehöhlung 6 umgrenzende Wandung des Kolbenstopfens 2 in die Innenhöhlung 34 des Schneidstempels 32 verdrängt wird. Dabei verkleinert sich der Querschnitt der Aufnahmehöhlung 6 entsprechend.

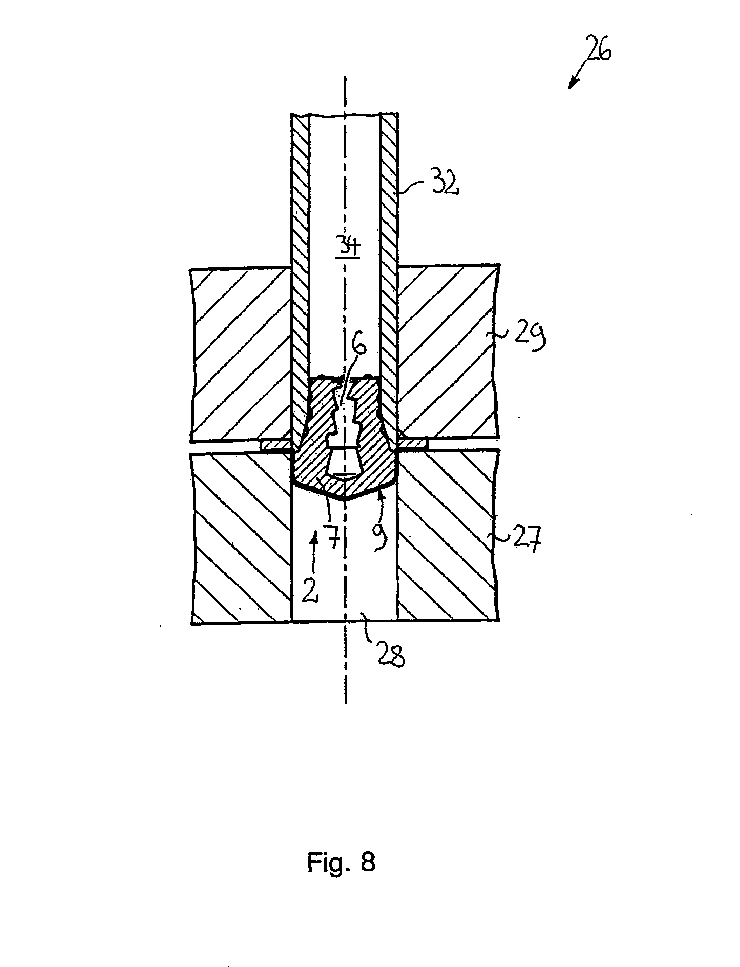

In Figur 8 ist erkennbar, daß der Durchmesser des Schneidstempels 32 etwa den Durchmesser der Öffnung 28 der Schneidmatrize 27 entspricht, so daß der Schichtanordnungsflansch 16 von dem Schneidstempel 32 bündig zu der in Gebrauchsstellung an dem Spritzenzylinder 1 anliegenden Teilbereich des Inertfilms 9 von dem Kolbenstopfen 2 abgetrennt wird. Dabei wird die Dichtzone 13 gebildet.

Die Erfindung betrifft also einen im wesentlichen aus Gummi bestehenden Kolbenstopfen 2, der einen in Gebrauchsstellung dem Inhalt eines Spritzen- oder Karpulenzylinders 1 zugewandten, von einem Inertfilm 9' kappenförmig umschlossenen Kolbenabschnitt 8 hat, der mit seinem Inertfilm 9' an dem Spritzen- oder Karpulenzylinder 1 anliegt. Benachbart zu diesem Kolbenabschnitt 8 hat der Kolbenstopfen 2 einen unbeschichteten Dichtabschnitt 10. Desweiteren bezieht sich die Erfindung auf eine Vorrichtung und ein Verfahren zum Herstellen eines solchen Kolbenstopfens 2. Ein unvulkanisiertes Gummifell 7 wird zusammen mit einem Inertfilm 9 in ein Formwerkzeug eingelegt und unter Druck- und Wärmeeinwirkung wird aus dieser Schichtanordnung die bis auf einen im Bereich der Trennebene des Formwerkzeugs seitlich über den Kolbenstopfen 2 überstehenden Flansch 16 vollständige Kolbenstopfenkontur gebildet. Eine Aufnahmehöhlung 6 für eine Kolbenstange wird bis in den von dem Inertfilm 9 kappenförmig umschlossenen Kolbenabschnitt 8 eingebracht. Der die Aufnahmehöhlung 6 umgenzende Wandungsbereich des Dichtabschnitts 10 wird in die Aufnahmehöhlung 6 verdrängt. Danach wird der Kolbenstopfen 2 durch einen Stanzvorgang von dem Flansch 16 abgetrennt, wobei in dem Dichtabschnitt 10 eine umlaufende, sich unmittelbar an den Rand des den Kolbenabschnitt 8 umschließenden Inertfilms 9' anschließende Dichtzone 13 gebildet wird.

## Patentansprüche

1. Verfahren zum Herstellen eines im wesentlichen aus Gummi oder dergleichen elastomerem Werkstoff bestehenden pharmazeutischen Kolbenstopfens (2), der eine Aufnahmehöhlung (6) zum Verbinden mit einer Kolbenstange (5) oder dergleichen Bewegungsübertragungselement und einen von einem fluorierten Polymerfilm oder dergleichen Inertfilm (9') kappenförmig umschlossenen Kolbenabschnitt (8) hat, der in Gebrauchsstellung dem Inhalt eines Spritzen- oder Karpulenzylinders (1) zugewandt ist und am Außenumfang mit seinem Inertfilm (9') an dem Spritzenoder Karpulenzylinder (1) anliegt, welcher Kolbenstopfen (2) benachbart zu diesem Kolbenabschnitt (8) einen unbeschichteten, in Gebrauchsstellung an dem Spritzenoder Karpulenzylinder (1) anliegenden Dichtabschnitt (10) hat, wobei in einem Formungsschritt eine aus einem unvulkanisierten Gummifell (7) und einem fluorierten Polymerfilm oder dergleichen Inertfilm (9) bestehende Schichtanordnung in ein Formwerkzeug eingelegt und unter Einwirkung von Druck und Wärme zu einem Kolbenstopfenteil geformt wird, wobei das Gummifell (7) vulkanisiert und untrennbar mit dem Inertfilm (9) verbunden wird, **dadurch gekennzeichnet, daß** während des Formungsschritts aus der Schichtanordnung die bis auf einen im Bereich der Trennebene des Formwerkzeugs verbleibende, seitlich über den Kolbenstopfen (2) überstehenden Schichtanordnungsflansch (16) vollständige Kontur des Kolbenstopfens (2) gebildet wird, daß die Aufnahmehöhlung (6) während des Formungsschritts bis über den Schichtanordnungsflansch (16) hinaus in den von dem Inertfilm (9) kappenförmig umschlossenen Kolbenabschnitt (8) in das Gummifell (7) eingebracht wird, daß in einem Abtrennvorgang zumindest der die Aufnahmehöhlung (6) umgenzende, den Schichtanordnungsflansch (16) überragende Wandungsbereich des Dichtabschnitts (10) in die Aufnahmehöhlung (6) verdrängt und der Kolbenstopfen (2) dann durch einen Stanzvorgang von dem Schichtanordnungsflansch (16) abgetrennt wird, so daß in dem Dichtabschnitt (10) eine ringförmig umlaufende, sich unmittelbar an Rand des den Kolbenabschnitt (8) kappenförmig umschließenden Inertfilms (9') anschließende Dichtzone (13) gebildet wird, die in Gebrauchsstellung an dem Spritzen- oder Karpulenzylinder (1) anliegt.

2. Verfahren nach Anspruch 1, **dadurch gekennzeichnet, daß** während des Formungsschritts wenigstens eine am Außenumfang des Dichtabschnitts (10) ringförmig umlaufende Dichtlippe (11) gebildet wird, und daß diese Dichtlippe (11) bei dem Abtrennvorgang zu der Aufnahmehöhlung (6) hin verdrängt wird.

3. Verfahren nach Anspruch 1 oder 2, **dadurch gekennzeichnet, daß** in dem Abtrennschritt der wenigstens eine von dem Inertfilm (9) kappenförmig umschlossene Kolbenabschnitt (8) zentrisch in eine Öffnung (28) einer Schneidmatrize (27) eingesetzt und der den Kolbenstopfen (2) umgrenzende Schichtanordnungsflansch (16) zwischen dieser Schneidmatrize (27) und einer Niederhalterplatte (29) eingespannt wird, wobei der den Schichtanordnungsflansch (16) überragende Teil des Dichtabschnitts (10) in eine in der Niederhalterplatte (29) vorgesehene Durchtrittsöffnung (30) eingreift, und daß danach der die Aufnahmehöhlung (6) des Kolbenstopfens (2) umgenzende Wandungsbereich des Dichtabschnitts (10) von einem Schneidstempel (32) in die Aufnahmehöhlung (6) verdrängt und der Schichtanordnungsflansch (16) von dem Kolbenstopfen (2) abgestanzt wird.

4. Verfahren nach einem der Ansprüche 1 bis 3, **dadurch gekennzeichnet, daß** der Schneidstempel (32) zum Abtrennen des Kolbenstopfens (2) von dem Schichtanordnungsflansch (16) von dem freien Ende des Dichtabschnitts (10) her auf den Schichtanordnungsflansch (16) zubewegt wird.

5. Verfahren nach einem der Ansprüche 1 bis 4, **dadurch gekennzeichnet, daß** das Gummifell (7) an seinem Außenrand umlaufend gegen wenigstens eine daran anliegende Formplatte (14, 15) des Formwerkzeugs abgedichtet wird und daß während und/oder nach dem Öffnen des Formwerkzeugs zum Ablösen der vulkanisierten Schichtanordnung von der Formplatte (14, 15) zwischen dieser und der Schichtanordnung ein Gas eingeblasen wird.

6. Verfahren nach einem der Ansprüche 1 bis 5, **dadurch gekennzeichnet, daß** zum Abdichten der vulkanisierten Schichtanordnung gegen die Formplatte (14, 15) während des Formungsschritts am Außenrand der Schichtanordnung eine um die Schichtanordnung umlaufende, an der Formplatte (14, 15) dichtend anliegende Dichtung (22) geformt wird.

7. Vorrichtung zur Herstellung von im wesentlichen aus Gummi oder dergleichen elastomerem Werkstoff bestehenden pharmazeutischen Kolbenstopfen (2), die eine Aufnahmehöhlung (6) zum Verbinden mit einer Kolbenstange (5) oder dergleichen Bewegungsübertragungselement und einen in Gebrauchsstellung dem Inhalt eines Spritzen- oder Karpulenzylinders (1) zugewandten, von einem fluorierten Polymerfilm oder dergleichen Inertfilm (9') kappenförmig umschlossenen Kolbenabschnitt (8) haben, der in Gebrauchsstellung am Außenumfang mit seinem Inertfilm (9') an dem Spritzen- oder Karpulenzylinder (1) anliegt, welcher Kolbenstopfen (2) benachbart zu diesem Kolbenabschnitt (8) einen unbeschichteten, in Gebrauchsstellung an dem Spritzen- oder Karpulenzylinder (1) anliegenden Dichtabschnitt (10) hat, zur Durchführung des Verfahrens nach einem der Ansprüche 1 bis 6, mit einem Formwerkzeug zum Formen des Kolbenstopfens (2) aus einer aus einem unvulkanisierten Gummifell (7) und einem fluorierten Polymerfilm oder dergleichen Inertfilm (9) bestehenden Schichtanordnung, wobei das Formwerkzeug zumindest eine erste Formplatte (14) und eine damit zusammenwirkende zweite Formplatte (15) hat, die relativ zueinander in eine Schließ- und eine Offenstellung bewegbar sind, sowie mit einer Stanzvorrichtung (26) zum Abtrennen eines im Bereich der Trennebene des Formwerkzeugs verbleibenden, seitlich über den Kolbenstopfen (2) überstehenden Schichtanordnungsflansches (16) von dem Kolbenstopfen (2), **dadurch gekennzeichnet, daß** das Formwerkzeug zur Formung der bis auf den im Bereich der Trennebene des Formwerkzeugs verbleibenden, seitlich über den Kolbenstopfen (2) überstehenden Schichtanordnungsflansch (16) vollständigen Kontur wenigstens eines Kolbenstopfens (2) ausgebildet ist und daß dazu die erste Formplatte (14) wenigstens eine erste, an den von dem Inertfilm (9) kappenförmig umschlossenen Kolbenabschnitt (8) des herzustellenden Kolbenstopfens (2) formangepaßte Kavität (17) und die zweite Formplatte (15) wenigstens eine zweite, an den unbeschichteten Dichtabschnitt (10) des Kolbenstopfens (2) formangepaßte Kavität (18) aufweist, daß in der zweiten Kavität (18) ein Formkern (20) zur Formung einer mit der Kolbenstange (5) oder dergleichen Bewegungsübertragungselement verbindbaren Aufnahmehöhlung (6) des Kolbenstopfens (2) angeordnet ist, der in Schließstellung des Formwerkzeugs mit seinem freien Ende in die gegenüberliegende erste Kavität (17) eingreift, daß die Stanzvorrichtung (26) eine Schneidmatrize (27) mit wenigstens einer Öffnung (28) zum zentrischen Einsetzen des von dem Inertfilm (9) kappenförmig umschlossenen Kolbenabschnitts (8) hat, daß der Schneidmatrize (27) zum Abtrennen des Schichtanordnungsflansches (16) von dem Kolbenstopfen (2) ein hülsenförmiger, an seinem Außenumfang eine Schneidkante aufweisender Schneidstempel (32) zugeordnet ist, der axial zur Öffnung (28) der Schneidmatrize (27) auf diese zu- und von dieser wegbewegbar ist, und daß die Innenhöhlung (34) des Schneidstempels (32) zur Aufnahme des nach innen verformten Dichtabschnitts (10) des Kolbenstopfens (2) ausgebildet ist.

8. Vorrichtung nach Anspruch 7, **dadurch gekennzeichnet, daß** die Stanzvorrichtung (26) eine Niederhalterplatte (29) mit zumindest einer zu der wenigstens einen Öffnung (28) der Schneidmatrize (27) fluchtenden Durchtrittsöffnung (30) zur Aufnahme des Dichtabschnitts (10) hat, daß die Niederhalterplatte (29) zum Einspannen des Schichtanordnungsflansches (16) zwischen ihr und der Schneidmatrize (27) axial zur Öffnung (28) der Schneidmatrize (27) relativ zu der Schneidmatrize (27) verschiebbar ist, und daß der Schneidstempel (32) in der Durchtrittsöffnung (30) der Niederhalterplatte (29) axial verschiebbar geführt ist.

9. Vorrichtung nach Anspruch 7 oder 8, **dadurch gekennzeichnet, daß** die Umfangswand der wenigstens einen Kavität (18) der zweiten Formplatte (15) zumindest eine ringörmig umlaufende Vertiefung (21) zur Bildung einer am Außenumfang des Kolbenstopfen-Dichtabschnitts ringförmig umlaufenden Dichtlippe (11) aufweist.

10. Vorrichtung nach einem der Ansprüche 7 bis 9, **dadurch gekennzeichnet, daß** der Schneidstempel (2) an seinem der Schneidmatrize (27) zugewandten freien Endbereich innenseitig eine Einführschräge (33) aufweist.

11. Vorrichtung nach einem der Ansprüche 7 bis 10, **dadurch gekennzeichnet, daß** der Außenrand der vulkanisierten Schichtanordnung mittels einer an der Schichtanordnung umlaufenden Dichtung (22) gegen wenigstens eine Formplatte (14, 15) abdichtbar ist, und daß in dem von der Dichtung (22) umgrenzten Bereich dieser Formplatte ein Gaszuführkanal (24) mündet, der zum Ablösen der Schichtanordnung von der Formplatte (14, 15) mit einer Gasdruckquelle verbindbar ist.

12. Vorrichtung nach einem der Ansprüche 7 bis 11, **dadurch gekennzeichnet, daß** wenigstens eine Formplatte (14, 15) zur Bildung der den Außenrand der Schichtanordnung gegen die Formplatte (14, 15) abdichtenden Dichtung (22) innerhalb ihrer Formhöhlung eine die Kavität(en) (17, 18) dieser Formplatte (14, 15) mit Abstand umgrenzende Ringnut (23) aufweist.

13. Vorrichtung nach einem der Ansprüche 7 bis 12, **dadurch gekennzeichnet, daß** in dem Mündungsbereich des Gaszuführkanals ein Ventil (25) angeordnet ist, vorzugsweise ein Tellerventil.

14. Pharmazeutischer Kolbenstopfen (2), der einen Grundkörper (7') aus Gummi oder dergleichen elastomerem Werkstoff aufweist, der eine Aufnahmehöhlung (6) zum Verbinden mit einer Kolbenstange (5) oder dergleichen Bewegungsübertragungselement und einen von einem fluorierten Polymerfilm oder dergleichen Inertfilm (9') kappenförmig umschlossenen Kolbenabschnitt (8) hat, der in Gebrauchsstellung dem Inhalt eines Spritzen- oder Karpulenzylinders (1) zugewandt ist und am Außenumfang mit seinem Inertfilm (9') an dem Spritzen- oder Karpulenzylinder (1) anliegt, wobei der Kolbenstopfen (2) benachbart zu diesem Kolbenabschnitt (8) einen unbeschichteten, in Gebrauchsstellung an dem Spritzen- oder Karpulenzylinder (1) anliegenden Dichtabschnitt (10) hat, der wenigstens eine an seinem Außenumfang umlaufende Dichtlippe (11) aufweist, **dadurch gekennzeichnet, daß** der Grundkörper (7') einstückig ausgebildet ist, daß sich die Aufnahmehöhlung (6) bis in den von dem Inertfilm (9') kappenförmig umschlossenen Kolbenabschnitt (8) erstreckt, und daß der Dichtabschnitt (10) an seinem Außenumfang eine ringförmig umlaufende, sich unmittelbar an den Rand des den Kolbenabschnitt (8) kappenförmig umschließenden Inertfilms (9') anschließende Dichtzone (13) aufweist, die in Gebrauchsstellung an dem Spritzen- oder Karpulenzylinder (1) flächig anliegt.

15. Pharmazeutischer Kolbenstopfen (2) nach Anspruch 14, **dadurch gekennzeichnet, daß** die Dichtzone (13) in gerader Verlängerung zu dem in Gebrauchsstellung an dem Spritzenoder Karpulenzylinder (1) anliegenden Außenumfangsabschnitt des Inertfilms (9') angeordnet ist oder radial etwas über diesen Außenumfangsabschnitt übersteht.

16. Pharmazeutischer Kolbenstopfen (2) nach Anspruch 14 oder 15, **dadurch gekennzeichnet, daß** ein Abschnitt (6a) der die Aufnahmehöhlung (6) umgrenzenden Innenwand des Kolbenstopfens (2) ein Innengewinde zum Verbinden mit dem Gewinde der Kolbenstange (5) oder dergleichen Bewegungsübertragungselement aufweist und daß dieser Innengewinde-Abschnitt mit Abstand vom Boden (6b) der Aufnahmehöhlung (6) endet.

17. Pharmazeutischer Kolbenstopfen (2) nach einem der Ansprüche 14 bis 16, **dadurch gekennzeichnet, daß** der Innengewinde-Abschnitt (6a) des Kolbenstopfens (2) an dem kappenförmig umschlossenen Kolbenabschnitt (8) endet oder von diesem beabstandet ist, und daß sich an den Innengewinde-Abschnitt (6a) vorzugsweise ein zylindrischer oder konischer Aufnahmehöhlungsabschnitt (6c) anschließt, der sich bis in den kappenförmig umschlossenen Kolbenabschnitt (8) erstreckt.

18. Pharmazeutischer Kolbenstopfen (2) nach einem der Ansprüche 14 bis 17, **dadurch gekennzeichnet, daß** sich der Querschnitt der Aufnahmehöhlung (6) ausgehend vom Boden (6b) zur Öffnung der Aufnahmehöhlung (6) hin konisch erweitert.

## Claims

1. Process for producing a pharmaceutical piston stopper (2) consisting substantially of rubber or a similar elastomeric material, which has a receiving cavity (6) for connection to a piston rod (5) or similar motion transmitting element and has a piston section (8) enclosed in the manner of a cap by a fluorinated polymer film or similar inert film (9'), which in the position of use faces the contents of a syringe or carpule cylinder (1) and at the outer periphery abuts with its inert film (9') on the syringe or carpule cylinder (1), the piston stopper (2) having, adjacent to this piston section (8), an uncoated sealing section (10) abutting on the syringe or carpule cylinder (1) in the position of use, while in a moulding step a layered arrangement consisting of an unvulcanised rubber sheet (7) and a fluorinated polymer film or similar inert film (9) is placed in a moulding tool and is moulded under the effect of pressure and heat into a rubber stopper component, the rubber sheet (7) being vulcanised and inseparably connected to the inert film (9), **characterised in that** during the moulding step the contour of the rubber stopper (2), which is complete apart from a flange (16) of the layered arrangement protruding laterally over the piston stopper (2) and remaining in the region of the plane of separation of the moulding tool, is formed from the layered arrangement, **in that** during the moulding step the receiving cavity (6) is formed in the rubber sheet (7) extending beyond the flange of the layered arrangement (16) into the piston section (8) surrounded in the manner of a cap by the inert film (9), **in that** in a separation process at least the part of the wall of the sealing section (10) which surrounds the receiving cavity (6) and projects over the flange of the layered arrangement (16) is displaced into the receiving cavity (6) and the piston stopper (2) is then separated from the flange of the layered arrangement (16) by a stamping process, so that a sealing zone (13) is formed in the sealing section (10), extending in an annular configuration and immediately adjacent to the edge of the inert film (9') which fits in the manner of a cap around the piston section (8), said sealing zone (13) abutting on the syringe or carpule cylinder in the position of use.

2. Process according to claim 1, **characterised in that** during the moulding step at least one sealing lip (11) is formed, running in a ring around the outer periphery of the sealing section (10) and **in that** this sealing lip (11) is displaced towards the receiving cavity (6) during the separation process.

3. Process according to claim 1 or 2, **characterised in that** in the separation step the minimum of one piston section (8) surrounded by the inert film (9) in the manner of a cap is inserted centrally in an opening (28) in a cutting matrix (27) and the layer mounted flange (16) surrounding the piston stopper (2) is braced between this cutting matrix (27) and a holding plate (29), while the part of the sealing section (10) which projects over the flange of the layered arrangement (16) engages in a through-opening (30) provided in the holding plate (29), and **in that** the wall region of the sealing section (10) surrounding the receiving cavity (6) of the piston stopper (2) is then displaced into the receiving cavity by a cutting stamp (32) and the flange of the layered arrangement (16) is separated from the piston stopper (2) by stamping.

4. Process according to one of claims 1 to 3, **characterised in that** in order to separate the piston stopper (2) from the flange of the layered arrangement (16) the cutting stamp (32) is moved from the free end of the sealing section (10) to the flange of the layered arrangement (16).

5. Process according to one of claims 1 to 4, **characterised in that** the rubber sheet (7) is sealed off around its outer edge from at least one moulding plate (14, 15) of the moulding tool abutting thereon and during and/or after the opening of the moulding tool to detach the vulcanised layered arrangement from the moulding plate (14, 15) a gas is blow in between said plate and the layered arrangement.

6. Process according to one of claims 1 to 5, **characterised in that** in order to seal the vulcanised layered arrangement off from the moulding plate (14, 15) during the moulding step a seal (22) is formed around the outer edge of the layered arrangement, which encircles the layered arrangement and fits closely against the moulding plate (14, 15).

7. Apparatus for producing pharmaceutical piston stoppers (2) consisting subsantially of rubber or a similar elastomeric material, which have a receiving cavity (6) for connection to a piston rod (5) or similar motion transmitting element and have a piston section (8) enclosed in the manner of a cap by a fluorinated polymer film or similar inert film (9'), which faces the contents of a syringe or carpule cylinder (1) in the position of use and at the outer periphery in the position of use abuts with its inert film (9') on the syringe or carpule cylinder (1), the piston stopper (2) having, adjacent to this piston section (8), an uncoated sealing section (10) abutting on the syringe or carpule cylinder (1) in the position of use, for carrying out the process according to one of claims 1 to 6, having a moulding tool for moulding the piston stopper (2) from a layered arrangement consisting of an unvulcanised rubber sheet (7) and a fluorinated polymer film or similar inert film (9), the moulding tool having at least a first moulding plate (14) and a second moulding plate (15) co-operating therewith, which are movable relative to one another into a closed position and an open position, and having a stamping device (26) for separating a flange (16) of the layered arrangement, remaining in the region of the plane of separation of the moulding tool and projecting laterally over the piston stopper (2), from the piston stopper (2), **characterised in that** the moulding tool is constructed to mould the contour of at least one rubber stopper (2) which is complete apart from the flange (16) protruding laterally over the piston stopper (2) and remaining in the region of the plane of separation of the moulding tool, and for this purpose the first moulding plate (14) has at least one first cavity (17) matched to the shape of the piston section (8) of the piston stopper (2) to be produced, which is surrounded by the inert film (9) in the form of a cap, and the second moulding plate (15) has at least a second cavity (18) matched to the shape of the uncoated sealing section (10) of the piston stopper (2), and in the second cavity (18) is provided a moulding core (20) for moulding a receiving cavity (6) of the piston stopper (2) which is connectable to the piston rod (5) or similar motion transmission element, which, in the closed position of the moulding tool, engages with its free end in the first cavity (17) opposite, **in that** the stamping device (26) has a cutting matrix (27) with at least one opening (28) for the central insertion of the piston section (8) surrounded by the inert film (9) in the manner of a cap, **in that** associated with the cutting matrix (27) for severing the flange (16) from the piston stopper (2) there is a sleeve-like cutting stamp having a cutting edge on its outer circumference which is axially movable towards and away from the opening (28) of the cutting matrix (27), and **in that** the inner cavity (34) of the cutting stamp (32) is adapted to accommodate the inwardly shaped sealing section (10) of the piston stopper (2).

8. Apparatus according to claim 7, **characterised in that** the stamping device (26) has a holding plate (29) with at least one through-opening (30) in alignment with the minimum of one opening (28) of the cutting matrix (27) for receiving the sealing section (10), **in that** for clamping the flange (16) the holding plate (29) is movable relative to the cutting matrix (27) between said flange (16) and the cutting matrix (27), axially with respect to the opening (28) of the cutting matrix (27), and **in that** the cutting stamp (32) is guided so as to be axially movable in the through-opening (30) in the holding plate (29).

9. Apparatus according to claim 7 or 8, **characterised in that** the circumferential wall of the minimum of one cavity (18) in the second moulding plate (15) has at least one annularly encircling depression (21) to form an annularly encircling sealing lip (11) on the outer periphery of the sealing section of the piston stopper.

10. Apparatus according to one of claims 7 to 9, **characterised in that** the cutting stamp (2) has an insertion slope (33) on the inside at its free end portion facing the cutting matrix (27).

11. Apparatus according to one of claims 7 to 10, **characterised in that** the outer edge of the vulcanised layer arrangement can be sealed off from at least one moulding plate (14, 15) by means of an encircling seal (22) on the layer arrangement, and **in that** a gas supply channel (24) opens into the region of this moulding plate bounded by the seal (22), this channel (24) being connectable to a gas pressure source in order to detach the layer arrangement from the moulding plate (14, 15).

12. Apparatus according to one of claims 7 to 11, **characterised in that** at least one moulding plate (14, 15) for forming the seal (22) which seals off the outer edge of the layered arrangement from the moulding plate (14, 15) has within its cavity an annular groove (23) which surrounds the cavity (or cavities) (17, 18) of this moulding plate (14, 15) at a spacing therefrom.

13. Apparatus according to one of claims 7 to 12, **characterised in that** a valve (25), preferably a plate valve, is arranged in the region of the opening of the gas supply channel.

14. Pharmaceutical piston stopper (2) which comprises a base member (7') of rubber or a similar elastomeric material, which has a receiving cavity (6) for connection to a piston rod (5) or similar motion transmitting element and has a piston section (8) enclosed in the manner of a cap by a fluorinated polymer film or similar inert film (9'), which in the position of use faces the contents of a syringe or carpule cylinder (1) and at the outer periphery abuts with its inert film (9') on the syringe or carpule cylinder (1), the piston stopper (2) having, adjacent to this piston section (8), an uncoated sealing section (10) abutting on the syringe or carpule cylinder (1) in the position of use, said sealing section having at least one sealing lip (11) running around its outer periphery, **characterised in that** the base member (7') is formed in one piece, the receiving cavity (6) extends into the piston section (8) surrounded by the inert film (9') in the manner of a cap, and **in that** the sealing section (10) comprises on its outer periphery an annularly extending sealing zone (13) immediately adjacent to the edge of the inert film (9') enclosing the piston section (8) in the manner of a cap, said sealing zone fitting closely against the syringe or carpule cylinder in the position of use.

15. Pharmaceutical piston stopper (2) according to claim 14, **characterised in that** the sealing zone (13) is arranged on a straight extension to the outer peripheral section of the inert film (9') which abuts on the syringe or carpule cylinder in the position of use, or projects radially somewhat beyond this outer peripheral section.

16. Pharmaceutical piston stopper (2) according to claim 14 or 15, **characterised in that** a section (6a) of the inner wall of the piston stopper (2) surrounding the receiving cavity (6) has an internal thread for connecting to the thread on the piston rod (5) or similar motion transmission element and **in that** this internally threaded section ends at a spacing from the base (6b) of the receiving cavity (6).

17. Pharmaceutical piston stopper (2) according to one of claims 14 to 16, **characterised in that** the internally threaded section (6a) of the piston stopper (2) ends at the piston section (8) surrounded in the manner of a cap or is at a spacing therefrom, and **in that** preferably adjoining the internally threaded section (6a) is a cylindrical or conical receiving cavity section (6c) which extends into the piston section (8) surrounded in the manner of a cap.

18. Pharmaceutical piston stopper (2) according to one of claims 14 to 17, **characterised in that** the cross section of the receiving cavity (6) widens out conically from the base (6b) to the opening of the receiving cavity (6).

## Revendications

1. Procédé de fabrication d'un bouchon-piston pharmaceutique (2), réalisé pour l'essentiel en caoutchouc ou matériau élastomère analogue, qui possède une cavité réceptrice (6) pour l'assemblage à une tige de piston (5) ou élément analogue de transmission de mouvement, et une partie de piston (8) entourée à la manière d'une coiffe par un film polymère fluoré ou film inerte analogue (9'), partie qui, en position d'utilisation, est tournée vers le contenu d'un cylindre (1) de seringue ou de cartouche et s'applique sur la périphérie extérieure par son film inerte (9') contre le cylindre (1) de seringue ou de cartouche, le bouchon-piston (2) possédant, au voisinage de cette partie de piston (8), une partie d'étanchéité non revêtue (10) s'appliquant en position d'utilisation contre le cylindre (1) de seringue ou de cartouche, procédé selon lequel, lors d'une étape de façonnage, un bloc stratifié constitué d'une feuille (7) de caoutchouc non vulcanisé et d'un film polymère fluoré ou film inerte analogue (9) est introduit dans un moule et façonné en un élément de bouchon-piston sous l'action de pression et de chaleur, la feuille de caoutchouc (7) étant alors vulcanisée et assemblée de manière inséparable au film inerte (9), **caractérisé en ce que**, pendant l'étape de façonnage à partir du bloc stratifié, on forme le contour complet du bouchon-piston (2), à l'exception d'une bride (16) de bloc stratifié restant présente dans la région du plan de séparation du moule en dépassant latéralement du bouchon-piston (2), **en ce que**, pendant l'étape de formage, la cavité réceptrice (6) est pratiquée dans la feuille de caoutchouc (7) jusque, au-delà de la bride (16) de bloc stratifié, dans la partie de piston (8) entourée à la manière d'une coiffe par le film inerte (9), **en ce que**, lors d'une opération de détachement, au moins la région de paroi de la partie d'étanchéité (10) qui circonscrit la cavité réceptrice (6) et s'élève plus haut que la bride (16) de bloc stratifié est refoulée dans la cavité réceptrice (6), et le bouchon-piston (2) est ensuite détaché de la bride (16) de bloc stratifié par une opération de découpage, de sorte qu'est formée, dans la partie d'étanchéité (10), une zone d'étanchéité (13) annulairement entourante se raccordant directement au bord du film inerte (9') entourant à la manière d'une coiffe la partie de piston (8), zone qui s'applique en position d'utilisation contre le cylindre (1) de seringue ou de cartouche.

2. Procédé selon la revendication 1, **caractérisé en ce que**, pendant l'étape de façonnage, on forme au moins une lèvre d'étanchéité (11) entourant annulairement la périphérie extérieure de la partie d'étanchéité (10), et **en ce que** cette lèvre d'étanchéité (11) est refoulée vers la cavité réceptrice (6) lors de l'opération de détachement.

3. Procédé selon la revendication 1 ou 2, **caractérisé en ce que**, lors de l'étape de détachement, la partie de piston (8) au moins unique, entourée à la manière d'une coiffe par le film inerte (9), est insérée de manière centrée dans une ouverture (28) d'une matrice de découpage (27), et la bride (16) de bloc stratifié circonscrivant le bouchon-piston (2) est serrée entre cette matrice de découpage (27) et une plaque de pressage (29), la région de la partie d'étanchéité (10) s'élevant plus haut que la bride (16) de bloc stratifié s'engageant dans une ouverture traversante (30) prévue dans la plaque de pressage (29), et **en ce que**, ensuite, la région de paroi de la partie d'étanchéité (10) circonscrivant la cavité réceptrice (6) du bouchon-piston (2) est refoulée par un poinçon de découpage (32) dans la cavité réceptrice (6), et la bride (16) de bloc stratifié est détachée du bouchon-piston (2) par découpage.

4. Procédé selon l'une des revendications 1 à 3, **caractérisé en ce que** le poinçon de découpage (32), afin de détacher le bouchon-piston (2) de la bride (16) de bloc stratifié, est déplacé depuis l'extrémité libre de la partie d'étanchéité (10) en direction de la bride (16) de bloc stratifié.

5. Procédé selon l'une des revendications 1 à 4, **caractérisé en ce que** la feuille de caoutchouc (7) est, sur son bord extérieur, étanchée tout autour vis-à-vis d'au moins une plaque de moule (14, 15) appliquée contre elle, et **en ce que**, pendant et/ou après l'ouverture du moule, un gaz est, afin de décoller le bloc stratifié vulcanisé de la plaque de moule (14, 15), insufflé entre cette plaque et le bloc stratifié.

6. Procédé selon l'une des revendications 1 à 5, **caractérisé en ce qu'**un joint d'étanchéité (22), s'appliquant en étanchéité contre la plaque de moule (14, 15) et entourant le bloc stratifié, est formé pendant l'étape de façonnage sur le bord extérieur du bloc stratifié afin d'étancher le bloc stratifié vulcanisé vis-à-vis de la plaque de moule (14, 15).

7. Dispositif de fabrication de bouchons-pistons pharmaceutiques (2), réalisés pour l'essentiel en caoutchouc ou matériau élastomère analogue, qui possèdent une cavité réceptrice (6) pour l'assemblage à une tige de piston (5) ou élément analogue de transmission de mouvement, et une partie de piston (8) entourée à la manière d'une coiffe par un film polymère fluoré ou film inerte analogue (9'), partie qui, en position d'utilisation, est tournée vers le contenu d'un cylindre (1) de seringue ou de cartouche et s'applique sur la périphérie extérieure par son film inerte (9') contre le cylindre (1) de seringue ou de cartouche, le bouchon-piston (2) possédant, au voisinage de cette partie de piston (8), une partie d'étanchéité non revêtue (10) s'appliquant en position d'utilisation contre le cylindre (1) de seringue ou de cartouche,
pour la mise en oeuvre du procédé selon l'une des revendications 1 à 6,
avec un moule pour façonner le bouchon-piston (2) à partir d'un bloc stratifié constitué d'une feuille (7) de caoutchouc non vulcanisé et d'un film polymère fluoré ou film inerte analogue (9), le moule possédant au moins une première plaque de moule (14) et une deuxième plaque de moule (15) coopérant avec la première, les plaques pouvant être déplacées l'une par rapport à l'autre dans une position fermée et une position ouverte, et avec un dispositif de découpage (26) pour détacher du bouchon-piston (2) une bride (16) de bloc stratifié restant présente dans la région du plan de séparation du moule en dépassant latéralement du bouchon-piston (2),
**caractérisé en ce que** le moule est conçu pour façonner le contour complet d'au moins un bouchon-piston (2), à l'exception de la bride (16) de bloc stratifié restant présente dans la région du plan de séparation du moule en dépassant latéralement du bouchon-piston (2), et **en ce que**, à cet effet, la première plaque de moule (14) présente au moins une première cavité (17) de forme adaptée à la partie de piston (8), entourée à la manière d'une coiffe par le film inerte (9), du bouchon-piston (2) à fabriquer, et la deuxième plaque de moule (15) présente au moins une deuxième cavité (18) de forme adaptée à la partie d'étanchéité non revêtue (10) du bouchon-piston (2), **en ce qu'**un noyau (20) est disposé dans la deuxième cavité (18) pour former une cavité réceptrice (6), pouvant être assemblée à la tige de piston (5) ou élément analogue de transmission de mouvement, du bouchon-piston (2), noyau qui, dans la position fermée du moule, s'engage par son extrémité libre dans la première cavité (17) opposée, **en ce que** le dispositif de découpage (26) possède une matrice de découpage (27) pourvue d'au moins une ouverture (28) pour l'insertion centrée de la partie de piston (8) entourée à la manière d'une coiffe par le film inerte (9), **en ce qu'**est associé à la matrice de découpage (27), afin de détacher la bride (16) de bloc stratifié du bouchon-piston (2), un poinçon de découpage (32) en forme de manchon, présentant un tranchant sur sa périphérie extérieure, poinçon qui peut être approché et éloigné axialement de l'ouverture (28) de la matrice de découpage (27), et **en ce que** la cavité intérieure (34) du poinçon de découpage (32) est conçue pour recevoir la partie d'étanchéité (10), déformée vers l'intérieur, du bouchon-piston (2).

8. Dispositif selon la revendication 7, **caractérisé en ce que** le dispositif de découpage (26) possède une plaque de pressage (29) pourvue d'au moins une ouverture traversante (30) alignée avec l'ouverture au moins unique (28) de la matrice de découpage (27), **en ce que** la plaque de pressage (29) peut être coulissée par rapport à la matrice de découpage (27), axialement à l'ouverture (28) de la matrice de découpage (27), afin de serrer la bride (16) de bloc stratifié entre ladite plaque de pressage (29) et la matrice de découpage (27), et **en ce que** le poinçon de découpage (32) est guidé en coulissement axial dans l'ouverture traversante (30) de la plaque de pressage (29).

9. Dispositif selon la revendication 7 ou 8, **caractérisé en ce que** la paroi périphérique de la cavité (18) au moins unique de la deuxième plaque de moule (15) présente au moins un renfoncement (21) annulairement entourant pour former une lèvre d'étanchéité (11) annulairement entourante sur la périphérie extérieure de la partie d'étanchéité du bouchon-piston.

10. Dispositif selon l'une des revendications 7 à 9, **caractérisé en ce que** le poinçon de découpage (32) présente un biais d'introduction (33) sur la face intérieure de sa région terminale libre tournée vers la matrice de découpage (27).

11. Dispositif selon l'une des revendications 7 à 10, **caractérisé en ce que** le bord extérieur du bloc stratifié vulcanisé peut être étanché vis-à-vis d'au moins une plaque de moule (14, 15) au moyen d'un joint d'étanchéité (22) entourant le bloc stratifié, et **en ce qu'**un canal d'alimentation en gaz (24) débouche dans la région de cette plaque de moule qui est circonscrite par le joint d'étanchéité (22), canal qui peut être relié à une source de gaz comprimé afin de décoller le bloc stratifié de la plaque de moule (14, 15).

12. Dispositif selon l'une des revendications 7 à 11, **caractérisé en ce qu'**au moins une plaque de moule (14, 15) présente à l'intérieur de sa cavité de moule, afin de former le joint d'étanchéité (22) étanchant le bord extérieur du bloc stratifié vis-à-vis de la plaque de moule (14, 15), une rainure annulaire (23) circonscrivant à distance la ou les cavités (17, 18) de cette plaque de moule (14, 15).

13. Dispositif selon l'une des revendications 7 à 12, **caractérisé en ce qu'**une soupape (25), de préférence une soupape à disque, est disposée dans la région d'embouchure du canal d'alimentation en gaz.

14. Bouchon-piston pharmaceutique (2), qui présente un corps de base (7') en caoutchouc ou matériau élastomère analogue, qui possède une cavité réceptrice (6) pour l'assemblage à une tige de piston (5) ou élément analogue de transmission de mouvement, et une partie de piston (8) entourée à la manière d'une coiffe par un film polymère fluoré ou film inerte analogue (9'), partie qui, en position d'utilisation, est tournée vers le contenu d'un cylindre (1) de seringue ou de cartouche et s'applique sur la périphérie extérieure par son film inerte (9') contre le cylindre (1) de seringue ou de cartouche, le bouchon-piston (2) possédant, au voisinage de cette partie de piston (8), une partie d'étanchéité non revêtue (10) s'appliquant en position d'utilisation contre le cylindre (1) de seringue ou de cartouche, partie qui présente au moins une lèvre d'étanchéité entourante (11) sur sa périphérie extérieure, **caractérisé en ce que** le corps de base (7') est réalisé d'un seul tenant, **en ce que** la cavité réceptrice (6) s'étend jusque dans la partie de piston (8) entourée à la manière d'une coiffe par le film inerte (9'), et **en ce que** la partie d'étanchéité (10) présente sur sa périphérie extérieure une zone d'étanchéité (13) annulairement entourante se raccordant directement au bord du film inerte (9') entourant à la manière d'une coiffe la partie de piston (8), zone qui, en position d'utilisation, s'applique à plat contre le cylindre (1) de seringue ou de cartouche.

15. Bouchon-piston pharmaceutique (2) selon la revendication 14, **caractérisé en ce que** la zone d'étanchéité (13) est disposée dans le prolongement rectiligne de la partie de la périphérie extérieure du film inerte (9') qui s'applique en position d'utilisation contre le cylindre (1) de seringue ou de cartouche, ou dépasse radialement quelque peu de cette partie de périphérie extérieure.

16. Bouchon-piston pharmaceutique (2) selon la revendication 14 ou 15, **caractérisé en ce qu'**une partie (6a) de la paroi intérieure du bouchon-piston (2) circonscrivant la cavité réceptrice (6) présente un filetage intérieur pour l'assemblage avec le filetage de la tige de piston (5) ou élément analogue de transmission de mouvement, et **en ce que** cette partie à filetage intérieur se termine à distance du fond (6b) de la cavité réceptrice (6).

17. Bouchon-piston pharmaceutique (2) selon l'une des revendications 14 à 16, **caractérisé en ce que** la partie (6a) à filetage intérieur du bouchon-piston (2) se termine au niveau de la partie de piston (8) entourée à la manière d'une coiffe ou en est distante, et **en ce que**, de préférence, une partie (6c) de cavité réceptrice cylindrique ou conique se raccorde à la partie (6a) à filetage intérieur et s'étend jusque dans la partie de piston (8) entourée à la manière d'une coiffe.

18. Bouchon-piston pharmaceutique (2) selon l'une des revendications 14 à 17, **caractérisé en ce que** la section de la cavité réceptrice (6) s'élargit coniquement depuis le fond (6b) en direction de l'ouverture de la cavité réceptrice (6).
